# EUROPEAN PATENT APPLICATION

(11) **EP 4 763 224 A1**
(43) Date of publication of application: **24.06.2026**
(21) Application number: 24854216.9
(22) Date of filing: 14.08.2024
(51) Int. Cl.: A61K 45/00, A61K 9/127, A61K 31/7032, A61P 35/00, A61P 35/04, A61P 43/00, C07H 15/04

(54) **ANTICANCER AGENT CONTAINING NKT CELL LIGAND-CONTAINING LIPOSOME COMPOSITION**

(30) Priority: 15.08.2023 JP 2023132368
(71) Applicant: RIKEN, Wako-shi, Saitama 351-0198 (JP); Peace Venture Capital 168 Co., Ltd., Tokyo 101-0047 (JP)
(72) Inventor: OHNO, Hiroshi, Wako-shi, Saitama 351-0198 (JP); TAIDA, Takashi, Wako-shi, Saitama 351-0198 (JP); JINNOHARA, Toshi, Wako-shi, Saitama 351-0198 (JP); TAKAHASHI, Masumi, Wako-shi, Saitama 351-0198 (JP); AMARI, Hirokuni, Tokyo 113-0034 (JP); KIKUCHI, Hiroshi, Tokyo 113-0034 (JP)
(74) Representative: HGF
(86) International application number: PCT/JP2024/028953
(87) International publication number: WO 2025/037627

(57) **Abstract**

(1) The purpose of the present invention is to provide an antitumor agent in which an effect of an immune checkpoint inhibitor is enhanced.
(2) The purpose of the present invention is also to provide a drug that could exhibit an effect even under conditions in which no antigen is present.

(1) Provided is an antitumor agent obtained by combining an immune checkpoint inhibitor with an NKT cell ligand-containing liposome composition.
(2) Also provided is a liposome composition containing an NKT cell ligand and an antigen.

## Description

### TECHNICAL FIELD

The present invention relates to:
(1) a pharmaceutical including a combination of an immune checkpoint inhibitor (e.g., an anti-PD-1 antibody) and a liposome composition containing an NKT cell ligand (e.g., a-GalCer, RK-163, RCAI-61, RCAI-137) capable of artificially activating NKT cells to exhibit antitumor activity in a cancer-bearing organism (a first mode of the present invention); and
(2) a liposome composition that includes an NKT cell ligand and an antigen and is capable of inducing long-term immune memory for prophylactic treatment for cancers or infections (a second mode of the present invention).

### BACKGROUND ART

Natural killer T (hereinafter referred to as NKT) cells are immune cells that were discovered to exhibit characteristics different from those of other lymphocyte lineages (T, B, and NK cells) and to belong to a new lymphocyte lineage (Non-Patent Document 1). NKT cells express a unique antigen receptor (Va24Ja18 (human), Va14Ja18 (mouse)), which makes them unique and completely distinguishable from other immune cell populations. They play an essential role not only in inducing activation of innate and acquired immune cell populations (Non-Patent Document 2) and enhancing antitumor effects (Non-Patent Documents 3, 4, and 5) but also in inducing long-term immune memory (Non-Patent Document 6).

The most important characteristic of NKT cells is that the unique antigen receptor expressed on NKT cells has an α chain (Va24Ja18 (human), Va14Ja18 (mouse)) that is activated by recognizing a glycolipid antigen bound to a CDld molecule, which is only one type per species and similar to major histocompatibility complex (MHC) class I (Non-Patent Documents 7 and 8). In other words, each species has only one type of NKT cell antigen receptor and CDld molecule, and their sequences are very similar between species. In terms of function, the mouse NKT cell antigen receptor can recognize the NKT cell ligand bound to human CD1d. The human NKT cell receptor can also recognize the NKT cell ligand bound to mouse CD1d. All humans share a common NKT cell receptor and a common CDld molecule. Thus, an NKT cell ligand-containing liposome composition may not only serve as a drug equally effective for all humans but also be usable for many types of mammals including dogs and cats.

α-Galactosylceramide (α-GalCer) was reported to strongly induce artificial activation of NKT cells (Non-Patent Document 9). Some compounds (e.g., RK-163, RCAI-61, RCAI-137) were also discovered having activity several tens times higher than that of α-galactosylceramide (Patent Documents 1 to 5 and Non-Patent Document 10). Together with other researchers, the inventors have developed a new NKT cell ligand-containing liposome composition that further contains such a high-activity compound (Patent Document 6 (International Application No. PCT/JP2023/005048 or WO2023/153527).

When administered in vivo, such a new NKT cell ligand-containing liposome composition will be taken up by antigen-presenting cells (APCs) so that the new NKT cell ligand can bind to the CDld molecule, which is similar to major histocompatibility complex (MHC) class I, and be presented on the antigen-presenting cell membrane. NKT cells can recognize the complex of the CDld molecule and the new NKT cell ligand presented on the antigen-presenting cells (in the case of human, using the α chain of the human NKT cell-unique antigen receptor Va24Ja18) and thus undergo artificial activation. The activated NKT cells can induce activation and rapid proliferation of a population of anticancer immune cells for enhancement of antitumor effects and induce long-term anticancer immune memory, which may suppress the progression, recurrence, or metastasis of cancer.

Upon pathogenesis, NKT cells, which normally reside in the liver, bone marrow, and lungs, can arrive at the lesion site before any other immune cells, although they make up only 0.01 to 0.1% of all white blood cells (very scarce in peripheral blood). This is because even when not activated, NKT cells can express CXCR6 (CXCL16 chemokine receptor) more strongly than any other cells and thus can rush to the lesion site when CXCL16, which can recruit NKT cells, is released from the lesion site into blood (Non-Patent Document 11). Consequently, NKT cells resident in the liver, bone marrow, and lungs can migrate toward CXCL16-rich regions and arrive at the lesion site before any other cells. At the time of arrival, however, NKT cells still remain unactivated. In the case where the lesion site is caused by infection of a pathogen, such as a bacterium, NKT cells having arrived at the lesion site can be immediately activated by an adjuvant substance from pathogen components. In the case of cancer, however, cancer cells, which are self-mutated cells having no adjuvant substance capable of activating NKT cells, cannot activate NKT cells, which means that NKT cells cannot induce antitumor enhancing effects or long-term anticancer immune memory and are ineffective against cancer cells. In this regard, if artificially activated, NKT cells may induce antitumor enhancing effects against cancer cells or induce long-term anticancer immune memory to prevent the development of cancer. In fact, some clinical trials using artificial activation of NKT cells to treat progressive lung cancer cases yielded a highly effective outcome (Non-Patent Document 12).

### Citation List

### Patent Documents

Patent Document 1: WO2008/102888
Patent Document 2: WO2009/119692
Patent Document 3: WO2010/030012
Patent Document 4: WO2011/096536
Patent Document 5: WO2013/162016
Patent Document 6: WO2023/153527

### Non-Patent Documents

Non-Patent Document 1: Proc. Natl. Acad. Sci. USA 1990, 87:5248-5252
Non-Patent Document 2: Nature Immunol 2003, 4: 1164-1165
Non-Patent Document 3: Cancer Sci 2008; 99: 638-645
Non-Patent Document 4: Science, 1997, 278, 1623-1626
Non-Patent Document 5: J Immunol 1999; 163: 2387-2391
Non-Patent Document 6: Front. Immunol., 2017, 25 September
Non-Patent Document 7: J. Immunol. 1998, 161, 3271-3281
Non-Patent Document 8: Cancer Res 1999; 59: 5102-5105
Non-Patent Document 9: Science, 1997, 278, 1626-1629
Non-Patent Document 10: Bioorganic & Medicinal Chemistry, 2013, 21: 3066-3079
Non-Patent Document 11: J Immunol., 2005, 175: 2051-2555
Non-Patent Document 12: J Immunol 2009, 182: 2492-2501
Non-Patent Document 13: Immunology 2012, 138:105-115
Non-Patent Document 14: J. Exp. Med., 2003, 198:267-279
Non-Patent Document 15: British J Cancer 2011, 105, 93-103
Non-Patent Document 16: Oncotarget 2018; 9:6201-6212
Non-Patent Document 17: Cytometry Research, 27(2): 39-44, 2017

### DISCLOSURE OF THE INVENTION

### Problems to be Solved by the Invention

Immune checkpoint inhibitors are becoming standard treatments for some refractory malignancies. Unfortunately, they produce an overall response rate of about 10 to 30% depending on cancer type and are completely ineffective for some patients. A reason for that may be that tumor tissues are in the state of immunodeficiency in which only a small subset of immune cells can be activated by cancer antigen stimulation to express PD-1 at the final stage. Studies have been actively conducted using a combination of two or more immune checkpoint inhibitors or a combination of an immune checkpoint inhibitor and any other anticancer agent, such as a chemotherapeutic agent, but unfortunately did not show the desired combinatorial effect (Non-Patent Document 17). Such studies do not always theoretically use a treatment for inducing the expression of PD-1 or its ligand (PD-L1) in combination with the immune checkpoint inhibitor. In addition, no drug has been developed to induce immune cell PD-1 expression in tumor tissues for accumulation of PD-1-positive immune cells in tumors or to induce strong expression of an immune checkpoint ligand molecule, such as a PD-L1 ligand molecule, on tumor cells for enhancement of the anti-PD-1 antibody. Thus, there is a need for the development of a drug for enhancing the efficacy of immune checkpoint inhibitors (a first problem).

NKT cell ligand-containing liposome compositions developed so far are designed to control cancer by artificially activating NKT cells in the body of cancer-bearing patients to produce an adjuvant effect, which can lead to further activation of a population of cancer-specific immune cells existing in the patients' body for enhancement of the antitumor function and lead to induction of long-term immune memory. Such compositions are used under conditions where cancer-bearing patients already have cancer antigens. Thus, there is a need for the development of a drug that is effective even in the absence of any cancer antigen (a second problem).

### Means for Solving the Problems

As a result of diligent research to provide a solution to the problem (first problem), the inventors have found that when artificially activated by administration of an NKT cell ligand-containing liposome, NKT cells can produce an IFNγ-mediated adjuvant effect that can lead to strong activation of a population of immune cells which would otherwise have remained not sufficiently activated in tumor tissues, lead to proliferation of such immune cells, and consequently lead to enhancement of PD-1 expression (see Test Example 1 shown later). The inventors have further found that the administration of an NKT cell ligand-containing liposome can enhance the expression of a PD-L1 ligand molecule on tumor cells (see Test Examples 2 to 4 shown later). In fact, the administration of a combination of an NKT cell ligand-containing liposome composition and an anti-PD-1 antibody to cancer-bearing animal models has been found to produce synergistic antitumor effects and to allow almost complete suppression of tumor growth as compared to the administration of each agent alone (see Test Example 5 shown later). Based on those new findings, the inventors have conducted further research and completed the present invention (a first mode of the present invention).

As a result of diligent research to provide a solution to the problem (second problem), the inventors have found that the administration of a liposome composition containing an NKT cell ligand and an antigen (e.g., a cancer antigen) can produce beneficial effects (e.g., antitumor effects) even in the absence of any antigen (e.g., any cancer antigen). Specifically, for example, upon administration, the liposome composition containing an NKT cell ligand and a cancer antigen can be taken by antigen-presenting cells so that the cancer antigen can be presented on subject-specific MHC HLA class I and II molecules to activate a population of cancer antigen-specific CD4T/CD8T cells. At the same time, the NKT cell ligand (the other component of the liposome composition) can also be presented together with the CDld molecule on antigen-presenting cells to induce artificial activation of NKT cells in vivo. The population of cancer antigen-specific T cells activated by the administered cancer antigen allows the artificially-activated NKT cells to produce adjuvant effects, which will lead to further enhanced activation followed by induction of long-term anticancer immune memory, so that a population of the resulting long-term immune cells can inhibit the development, recurrence, or metastasis of cancer. Based on those new findings, the inventors have conducted further research and completed the present invention (a second mode of the present invention).

Specifically, the present invention is directed to the following aspects.
[1] An antitumor agent including a combination of an immune checkpoint inhibitor and an NKT cell ligand-containing liposome composition.
[2] The antitumor agent according to aspect [1], wherein the NKT cell ligand is a glycolipid selected from the group consisting of a compound represented by Formula (I) below, a compound represented by Formula (II) below, and salts of the foregoing compounds, 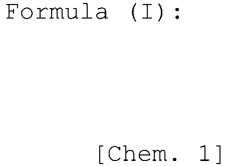 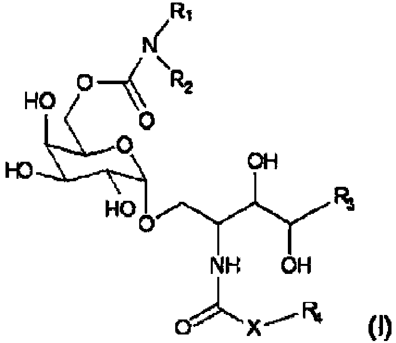
   wherein X represents an alkylene group or -NH-,
      R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with the adjacent nitrogen atom,
      R₃ represents a hydrocarbon group having 1 to 20 carbon atoms, and
      R₄ represents a hydrocarbon group having 1 to 30 carbon atoms, 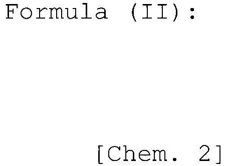 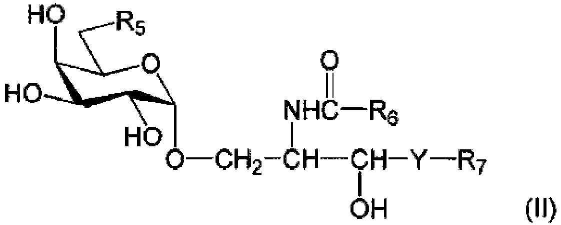
   wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.
[3] The antitumor agent according to aspect [2], wherein the glycolipid is the compound represented by Formula (I) or a salt thereof.
[4] The antitumor agent according to aspect [3], wherein the glycolipid is a compound represented by the formula: 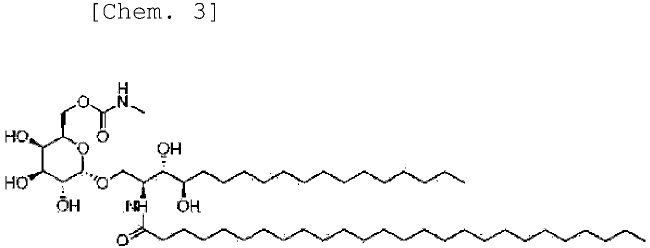 or a salt thereof.
[5] An agent for enhancing immune checkpoint molecule expression by activating a population of anticancer immune cells, the agent including an NKT cell ligand-containing liposome composition.
[6] An agent for enhancing expression of an immune checkpoint ligand molecule on a cancer cell by artificially activating NKT cells, the agent including an NKT cell ligand-containing liposome composition.
[7] A composition for enhancing the therapeutic effect of an immune checkpoint inhibitor, the composition including an NKT cell ligand-containing liposome.
[8] An agent for enhancing the therapeutic effect of an NKT ligand-containing liposome composition, the agent including an immune checkpoint inhibitor.
[9] A method of treating a malignant tumor in a subject, the method including administering an immune checkpoint inhibitor and an NKT cell ligand-containing liposome composition to the subject.
[10] The method according to aspect [9], wherein the NKT cell ligand is a glycolipid selected from the group consisting of a compound represented by Formula (I) below, a compound represented by Formula (II) below, and salts of the foregoing compounds, 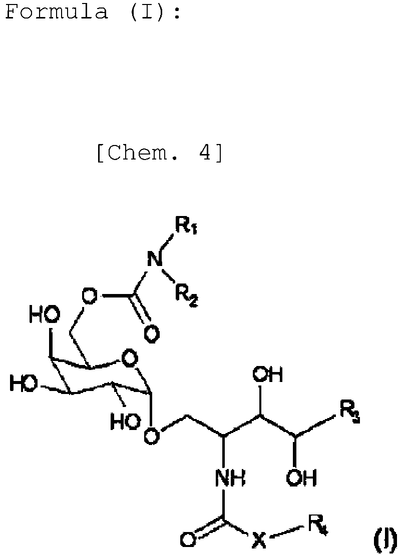
   wherein X represents an alkylene group or -NH-,
      R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with the adjacent nitrogen atom,
      R₃ represents a hydrocarbon group having 1 to 20 carbon atoms, and
      R₄ represents a hydrocarbon group having 1 to 30 carbon atoms, 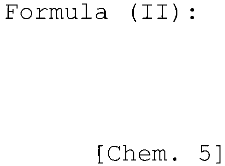 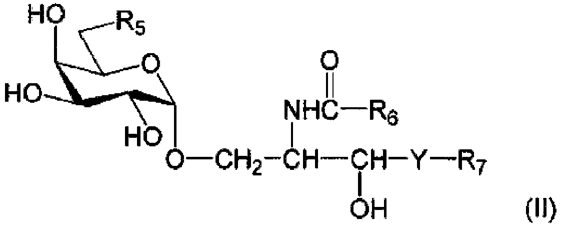
   wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.
[11] The method according to aspect [10], wherein the glycolipid is the compound represented by Formula (I) or a salt thereof.
[12] The method according to aspect [11], wherein the glycolipid is a compound represented by the formula: 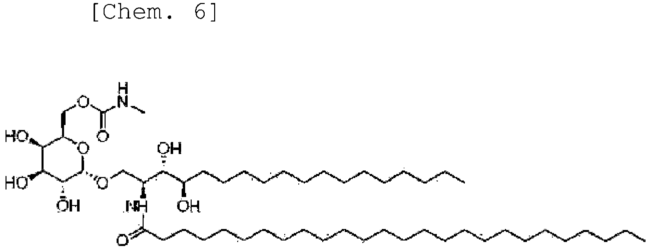 or a salt thereof.
[13] A method of enhancing immune checkpoint molecule expression in a subject, the method including administering, to the subject, an NKT cell ligand-containing liposome composition to activate a population of anticancer immune cells.
[14] A method of enhancing expression of an immune checkpoint ligand molecule on a cancer cell in a subject, the method including administering, to the subject, an NKT cell ligand-containing liposome composition to artificially activate NKT cells in the subject.
[15] A pharmaceutical for use in malignant tumor treatment, the pharmaceutical including a combination of an immune checkpoint inhibitor and an NKT cell ligand-containing liposome composition.
[16] The pharmaceutical according to aspect [15], wherein the NKT cell ligand is a glycolipid selected from the group consisting of a compound represented by Formula (I) below, a compound represented by Formula (II) below, and salts of the foregoing compounds, 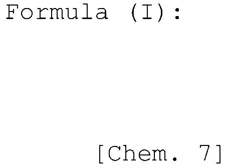 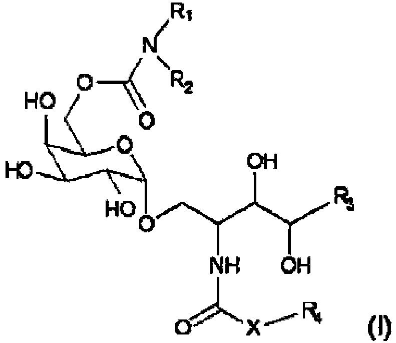
   wherein X represents an alkylene group or -NH-,
      R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with the adjacent nitrogen atom,
      R₃ represents a hydrocarbon group having 1 to 20 carbon atoms, and
      R₄ represents a hydrocarbon group having 1 to 30 carbon atoms, 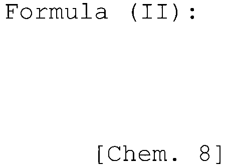 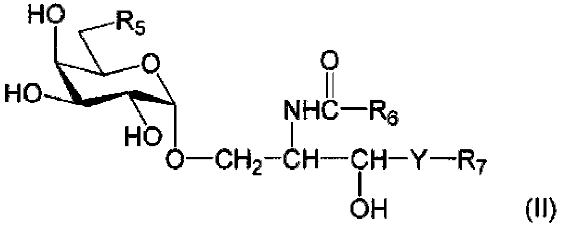
   [0032] wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.
[17] The pharmaceutical according to aspect [16], wherein the glycolipid is the compound represented by Formula (I) or a salt thereof.
[18] The pharmaceutical according to aspect [17], wherein the glycolipid is a compound represented by the formula: 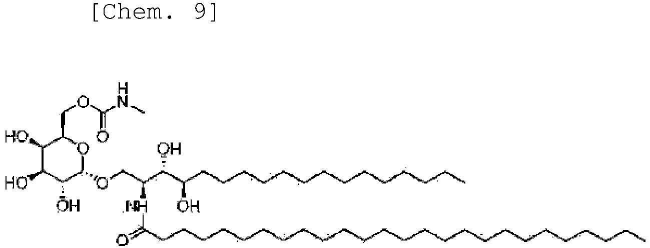 or a salt thereof.
[19] A composition for use in enhancing immune checkpoint molecule expression by activating a population of anticancer immune cells, the composition including an NKT cell ligand-containing liposome.
[20] A composition for use in enhancing expression of an immune checkpoint ligand molecule on a cancer cell by artificially activating NKT cells, the composition including an NKT cell ligand-containing liposome.
[21] A composition for use in enhancing the therapeutic effect of an immune checkpoint inhibitor, the composition including an NKT cell ligand-containing liposome.
[22] An agent for use in enhancing the therapeutic effect of an NKT cell ligand-containing liposome composition, the agent including an immune checkpoint inhibitor.
[23] A composition for use as a pharmaceutical, the composition including: an immune checkpoint inhibitor; and an NKT cell ligand-containing liposome.
[24] Use of a combination of an immune checkpoint inhibitor and an NKT cell ligand-containing liposome composition for the manufacture of an antitumor agent.
[25] The use according to aspect [24], wherein the NKT cell ligand is a glycolipid selected from the group consisting of a compound represented by Formula (I) below, a compound represented by Formula (II) below, and salts of the foregoing compounds, 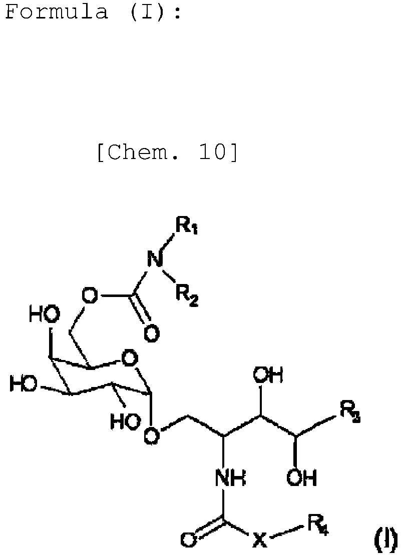
   wherein X represents an alkylene group or -NH-,
      R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with the adjacent nitrogen atom,
      R₃ represents a hydrocarbon group having 1 to 20 carbon atoms, and
      R₄ represents a hydrocarbon group having 1 to 30 carbon atoms, 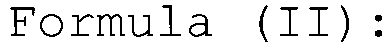 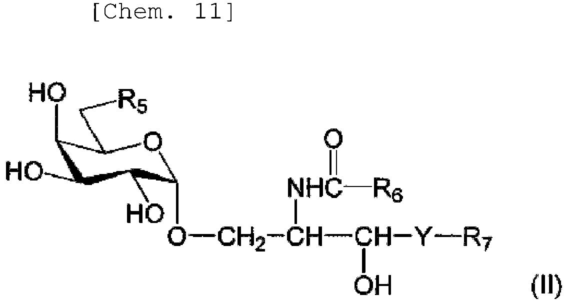
   wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.
[26] The use according to aspect [25], wherein the glycolipid is the compound represented by Formula (I) or a salt thereof.
[27] The use according to aspect [26], wherein the glycolipid is a compound represented by the formula: 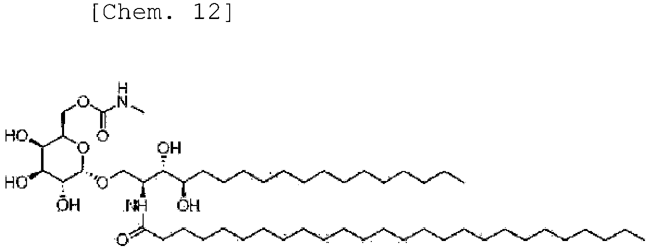 or a salt thereof.
[28] Use of an NKT cell ligand-containing liposome composition for the manufacture of an agent for enhancing immune checkpoint molecule expression by activating a population of anticancer immune cells.
[29] Use of an NKT cell ligand-containing liposome composition for the manufacture of an agent for enhancing expression of an immune checkpoint ligand molecule on a cancer cell by artificially activating NKT cells.
[30] Use of an NKT cell ligand-containing liposome composition for the manufacture of an agent for enhancing the therapeutic effect of an immune checkpoint inhibitor.
[31] Use of an immune checkpoint inhibitor for the manufacture of an agent for enhancing the therapeutic effect of an NKT cell ligand-containing liposome composition.
[32] A pharmaceutical composition including a combination of an immune checkpoint inhibitor and an NKT cell ligand-containing liposome composition.
[33] A liposome composition including: an NKT cell ligand; and an antigen.
[34] The composition according to aspect [33], wherein the NKT cell ligand is a glycolipid selected from the group consisting of a compound represented by Formula (I) below, a compound represented by Formula (II) below, and salts of the foregoing compounds, 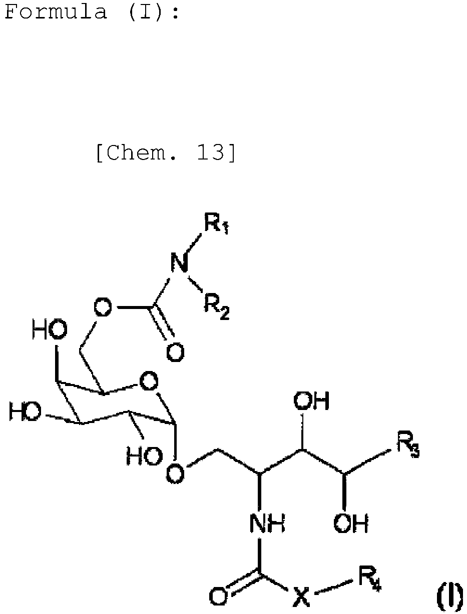
   wherein X represents an alkylene group or -NH-,
      R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with the adjacent nitrogen atom,
      R₃ represents a hydrocarbon group having 1 to 20 carbon atoms, and
      R₄ represents a hydrocarbon group having 1 to 30 carbon atoms, 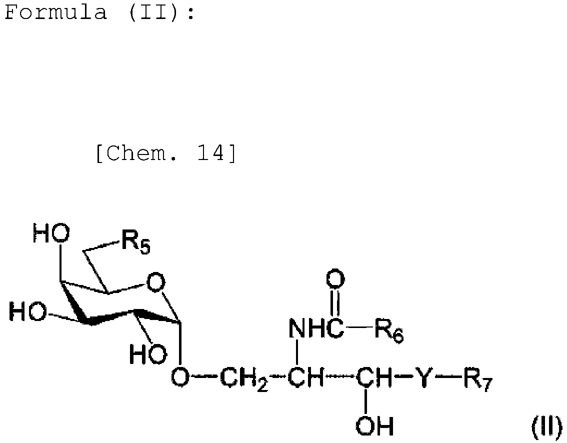
   [0046] wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.
[35] The composition according to aspect [34], wherein the glycolipid is the compound represented by Formula (I) or a salt thereof.
[36] The composition according to aspect [35], wherein the glycolipid is a compound represented by the formula: 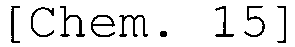 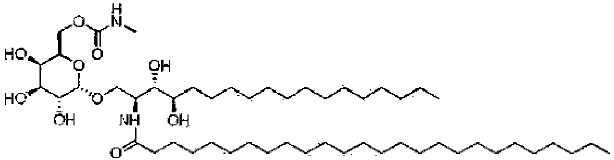 or a salt thereof.
[37] The composition according to any one of aspects [33] to [36], wherein the composition is for use in inducing immune memory.
[38] The composition according to any one of aspects [33] to [36], wherein the antigen is a cancer antigen.
[39] The composition according to aspect [38], wherein the composition is for use in prophylactically inducing, even in the absence of cancer, activation and proliferation of a population of immune cells capable of recognizing a cancer antigen.
[40] The composition according to aspect [38], wherein the composition is for use in prophylactically inducing anticancer immune memory even in the absence of cancer.
[41] The composition according to aspect [38], wherein the composition is for use in providing an enhanced preventive effect against development, recurrence, and metastasis of cancer.
[42] A method of inducing immune memory in a subject, the method including administering the composition according to any one of aspects [33] to [36] to the subject.
[43] A method of prophylactically inducing, even in the absence of cancer, activation and proliferation of a population of immune cells capable of recognizing a cancer antigen in a subject, the method including administering the composition according to aspect [38] to the subject.
[44] A method of prophylactically inducing anticancer immune memory even in the absence of cancer in a subject, the method including administering the composition according to aspect [38] to the subject.
[45] A method of inhibiting development, recurrence, and metastasis of cancer in a subject, the method including administering the composition according to aspect [38] to the subject.
[46] The composition according to any one of aspects [33] to [36], wherein the composition is for use in inducing immune memory.
[47] The composition according to aspect [38], wherein the composition is for use in prophylactically inducing, even in the absence of cancer, activation and proliferation of a population of immune cells capable of recognizing a cancer antigen.
[48] The composition according to aspect [38], wherein the composition is for use in prophylactically inducing anticancer immune memory even in the absence of cancer.
[49] The composition according to aspect [38], wherein the composition is for use in providing an enhanced preventive effect against development, recurrence, and metastasis of cancer.
[50] The composition according to any one of aspects [33] to [36], wherein the composition is for use as a pharmaceutical.
[51] Use of the composition according to any one of aspects [33] to [36] for the manufacture of an immune memory inducing agent.
[52] Use of the composition according to aspect [38] for the manufacture of an agent for prophylactically inducing, even in the absence of cancer, activation and proliferation of a population of immune cells capable of recognizing a cancer antigen.
[53] Use of the composition according to aspect [38] for the manufacture of an agent for prophylactically inducing anticancer immune memory even in the absence of cancer.
[54] Use of the composition according to aspect [38] for the manufacture of an agent for inhibiting development, recurrence, and metastasis of cancer.

### Effects of the Invention

The present invention (the first mode) is directed to a combination of an NKT cell ligand-containing liposome composition and an immune checkpoint inhibitor. Such a combination can be a useful therapeutic agent for the treatment of cancer or for the treatment of cancer metastasis or recurrence.

The NKT cell ligand-containing liposome can activate a population of anticancer immune cells accumulating in tumor tissues in a cancer-bearing organism, which may result in strong expression of an immune checkpoint molecule, such as a PD-1 molecule, and enhancement of the expression of an immune checkpoint ligand molecule, such as PD-L1, on tumor cells. On the basis of the research described above, the combination of an NKT cell ligand-containing liposome and an immune checkpoint inhibitor (according to the first mode) can be administered to artificially activate NKT cells in an organism. Such artificial activation may result in activation of an anticancer immune system and enhancement of the expression of an immune checkpoint ligand molecule (e.g., PD-L1) on tumor cells, which may consequently enhance the inhibitory effect of the immune checkpoint inhibitor to induce strong antitumor effects and to allow for powerful treatment of cancer. Such a combination therapy may also have stronger antitumor effects against a certain type of cancer that would otherwise have been resistant to monotherapy with an immune checkpoint inhibitor or an antitumor agent due to weak expression of the immune checkpoint ligand molecule.

PD-1 is a receptor molecule belonging to the CD28 family (a group of molecules that help to positively or negatively regulate T cell activation) expressed on activated lymphocytes (e.g., CD4 T cells and CD8 T cells of the acquired immune system and NK cells of the innate immune system). By binding to the PD-1 ligand (PD-L1 and PD-L2) expressed on cancer cells, PD-1 transmits inhibitory signals to a population of activated lymphocytes (CD4T, CD8T, NK) to negatively regulate the state of activation of the lymphocytes, which can inhibit the function of the population of activated lymphocytes and help the survival of cancer cells. The PD-1/PD-L1 pathway (immune checkpoint pathway) is essentially a so-called fail-safe mechanism to prevent the immune system from attacking self-cells. Unfortunately, cancer cells, which are also self-cells, are considered to use such a pathway as one of mechanisms for escaping the attack by the immune system and so on. Various immune checkpoint inhibitors, including anti-PD-1 antibodies and anti-PD-1 ligand antibodies, have been developed and clinically applied for long-term maintenance and enhancement of the function of a population of activated lymphocytes against tumors and for enhancement of attack to cancer cells. When used in combination with an immune checkpoint inhibitor, the NKT cell ligand-containing liposome composition can artificially activate NKT cells to induce activation and proliferation of a population of cancer-attacking lymphocytes and to induce strong expression of PD-L1 (PD-1 ligand) on tumor cells and thereby to enhance the effect of the immune checkpoint inhibitor, which may be expected to produce stronger anticancer effects. The population of cancer-attacking lymphocytes (TILs) accumulating in tumors is known to correlate with patient prognosis (Non-Patent Document 15). Some reported data also shows that tumor infiltration of TILs including more PD-1-positive lymphocytes resulted in better prognosis (Non-Patent Document 16). The anti-PD-1 antibody, which is an immune checkpoint inhibitor first approved for production and marketing, is becoming a standard treatment for progressive cancer since it has been currently confirmed to be more effective in reducing tumor size and extending lifetime than chemotherapy for various types of cancer, such as melanoma, non-small cell lung cancer, renal cell carcinoma, Hodgkin lymphoma, and head and neck cancer. However, it has been reported to be ineffective against some types of cancer (Non-Patent Document 16).

Therefore, the combination of the NKT cell ligand-containing liposome and the anti-PD-1 antibody (according to the first mode) is expected not only to have stronger anticancer effects than they do alone but also to be effective against a certain type of cancer resistant to monotherapy with the anti-PD-1 antibody because the combination can induce artificial activation of NKT cells to enhance the expression of PD-L1 on cancer cells and thus to allow the anti-PD-1 antibody to have stronger antitumor effects (the first mode of the present invention).

The in vivo administration of the NKT cell ligand-containing liposome composition will also be expected to have the effects described below.
(1) The NKT cell ligand can artificially activate NKT cells, which can then rapidly produce a large amount of Th1 cytokines, such as interferon-y. The Th1 cytokines can provide an adjuvant effect to strongly induce the expression of a checkpoint ligand molecule (e.g., PD-L1) on a cancer cell membrane for self-protection against the attack by an immune cell population.
(2) At the same time, the NKT cell ligand can induce activation and rapid proliferation of NK cells, CD4 T cells, CD8 T cells, γδ T cells, and other members of the effector immune cell population (Non-Patent Document 2) to provide enhanced antitumor effects and thus to allow the immune cell population to strongly express PD-1.
(3) This may result in activation of a population of anticancer immune cells to provide enhanced antitumor effects and may result in induction of long-term anticancer immune memory over a period as long as one year, which may allow the attack to continue and to suppress the progression, recurrence, and metastasis of cancer (Non-Patent Document 6).
(4) In addition to the activation of a population of other immune cells, the NKT cells can express various cell-killing inducers, such as perforin, to exhibit direct cytotoxicity against target cells (Non-Patent Document 8).
(5) Their cytotoxicity can function to directly kill immunosuppressive macrophages (MDSCs) and tumor-associated macrophages (TAMs) induced by cancer cells to protect themselves from immune attack, which may lead to the improvement of the immune environment in cancer tissues (Non-Patent Document 13).
(6) In the immune system, only the NKT cells can manage the interaction between the NKT call ligand and immature dendric cells with no antigen-presenting capacity and can induce the differentiation of immature dendritic cells into mature ones and improve the immune environment in cancer tissues (Non-Patent Document 14).

The present invention (the second mode) is also directed to a liposome composition including a cancer antigen and an NKT cell ligand capable of inducing artificial activation of NKT cells. Such a liposome composition can be a useful antitumor vaccine agent for prevention of the development, recurrence, or metastasis of cancer. The cancer antigen may preferentially include a WT-1 antigen peptide, which is expressed in almost all carcinomas, or may include any other peptide having a sequence characteristic of carcinomas. Instead of the cancer antigen, the liposome composition may include a pathogen antigen. In such a case, the liposome composition can also be an infection-preventing agent (vaccine).

The NKT cell ligand-containing liposome composition is a preparation developed for the treatment of cancer-bearing subjects having cancer cells and cancer antigens. On the other hand, the liposome composition including an NKT cell ligand and a cancer antigen (according to the second mode) has been developed to address normal or cancer-free conditions and to be effective in preventing the development, recurrence, or metastasis of cancer. Examples of in vivo conditions with no cancer antigen include normal conditions, conditions with potential tumor residue or potential micrometastasis after tumor resection, conditions with a risk of developing heritable or familial cancer, and conditions with an increase in tumor marker level but with no detectable cancer lesion. The liposome composition including an NKT cell ligand and a cancer antigen (according to the second mode) may be effective for conditions with no or very less cancer antigen but with a concern about rapid cancer growth.

The liposome composition (according to the second mode) can supply the NKT cell ligand and the antigen (e.g., cancer antigen) simultaneously. The liposome composition may have the NKT cell ligand on the surface of the NKT cell-containing liposome and have the antigen (e.g., cancer antigen) encapsuled in the liposome. Specifically, the liposome composition may have the cancer antigen encapsulated in the above-mentioned NKT cell ligand-containing liposome composition (International Application No. PCT/JP2023/005048 or WO2023/153527). Thus, for example, the liposome composition including the NKT cell ligand and the antigen (according to the second mode) can be most effective,when the antigen is a cancer antigen, (1) at a stage before the development of familial cancer that should be prevented, (2) for the prevention of cancer recurrence after cancer resection and disappearance of cancer antigens, or (3) for the prophylactic treatment of a subject with a significant increase in tumor marker level but with no detectable cancer lesion. Such a subject with near-normal conditions may have a population of T cells capable of recognizing cancer antigens. Unfortunately, each of such T cells should have a small clone size and cannot be expected to have anticancer effects. In addition, during the cancer-bearing period, such a subject should have had neither established long-term cancer immune memory nor a sufficient number of immune cells capable of suppressing cancer development. However, the subject with such conditions can undergo the treatment for increasing a population of cancer-specific immune cells and inducing long-term cancer immune memory to have an immune system against cancer development or recurrence.

### BRIEF DESCRIPTION OF THE DRAWINGS

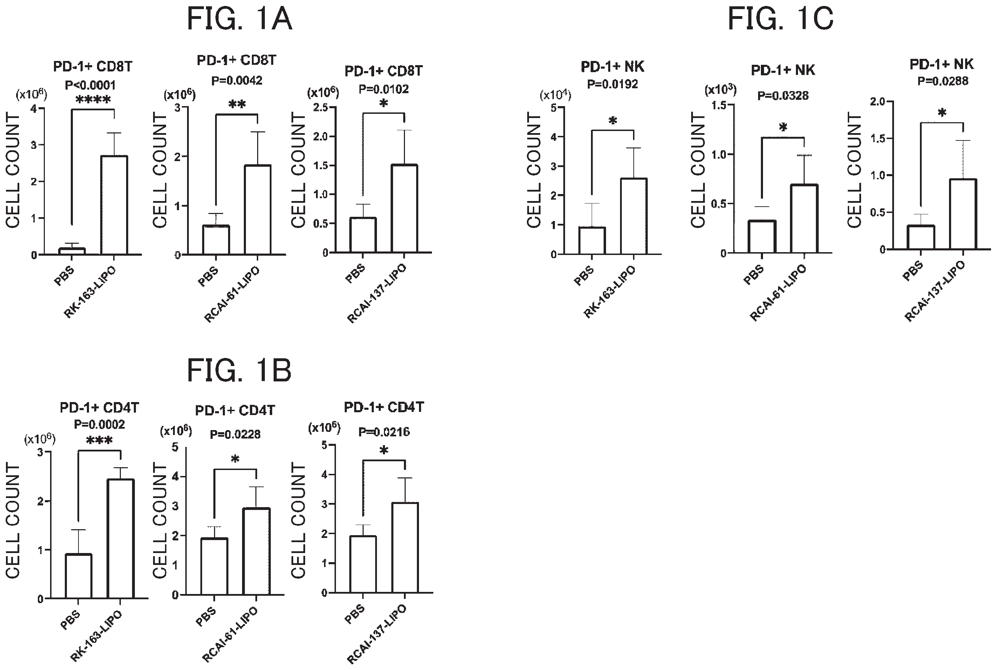

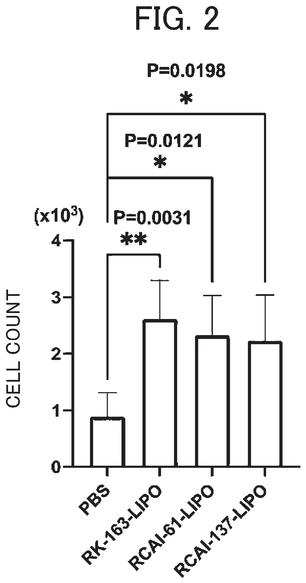

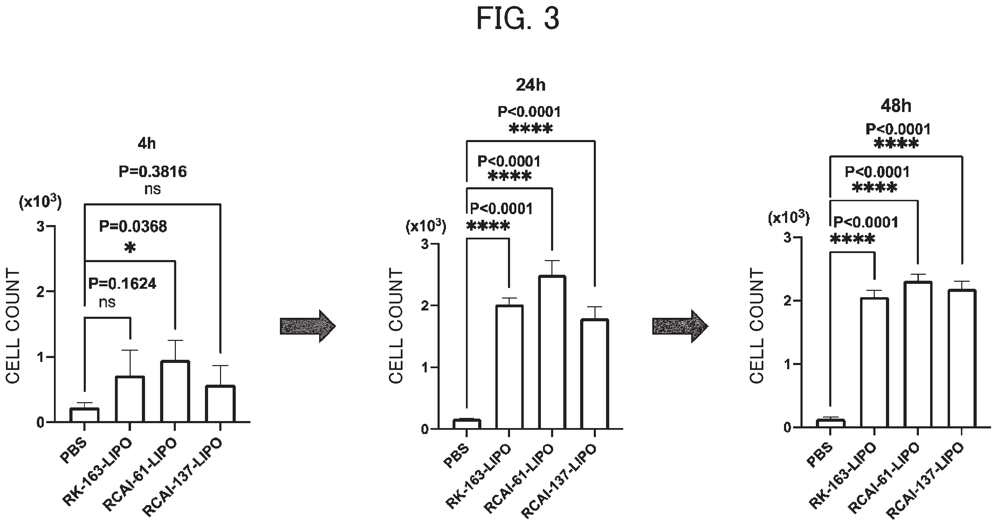

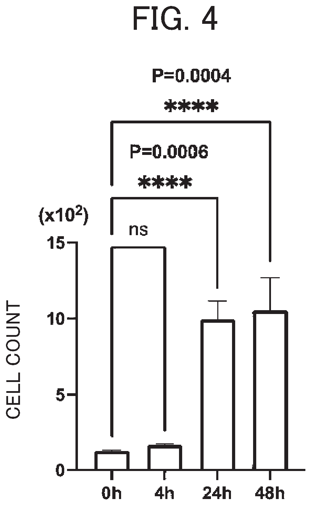

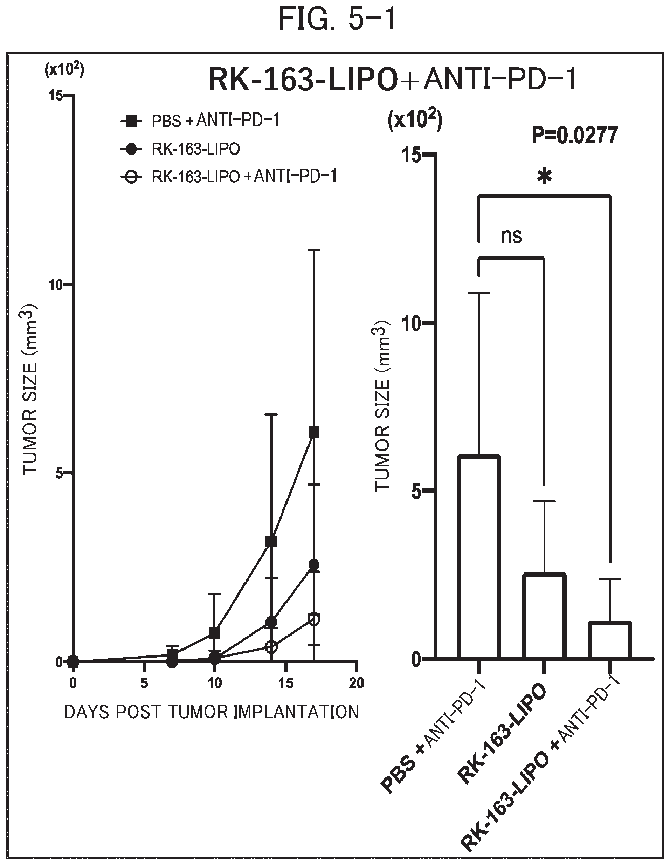

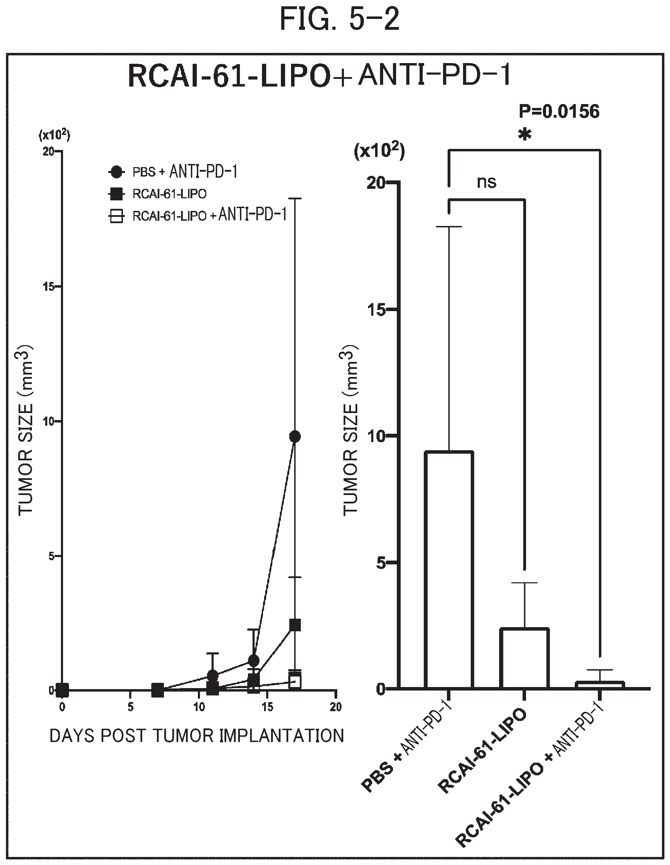

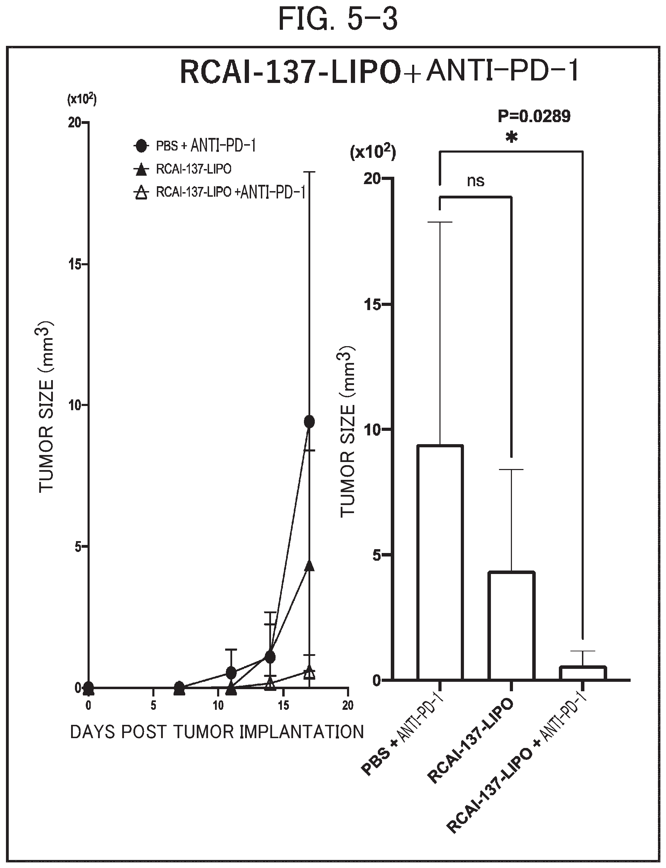

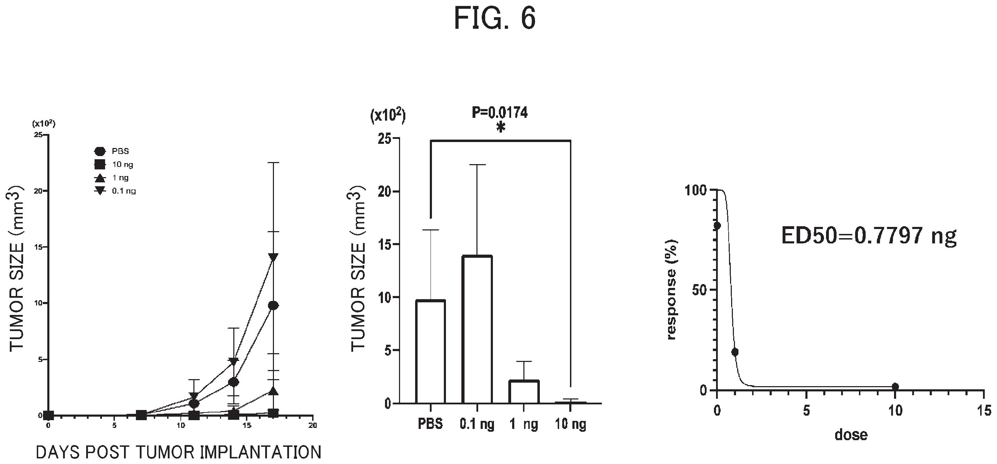

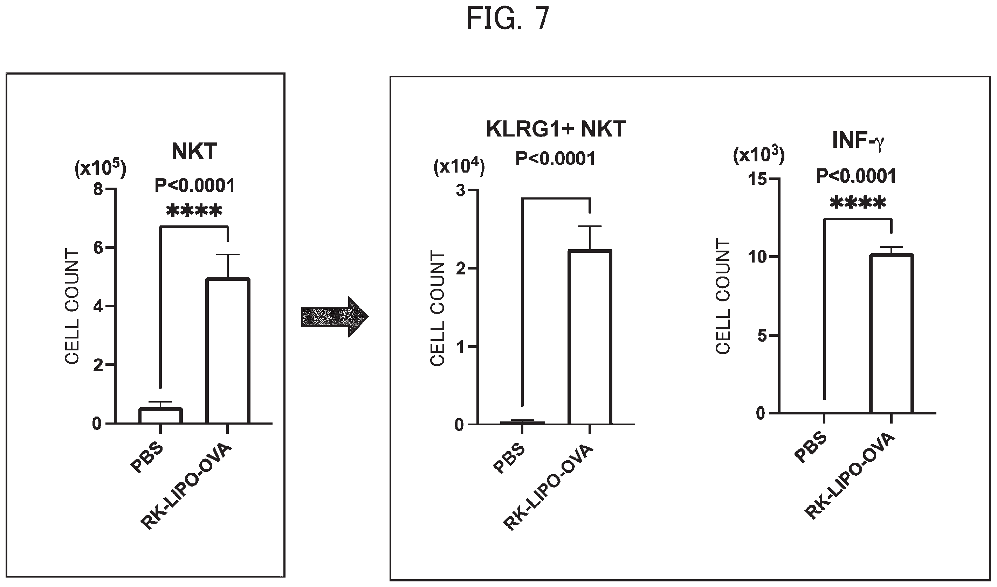

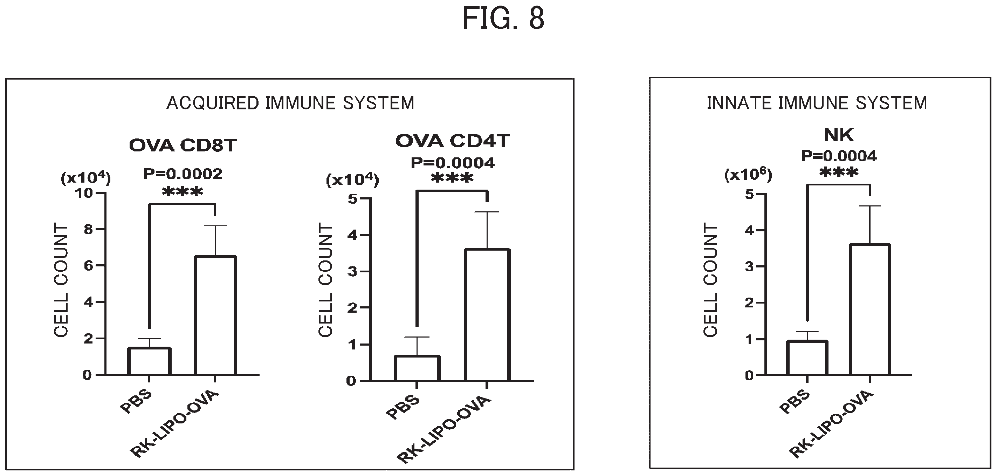

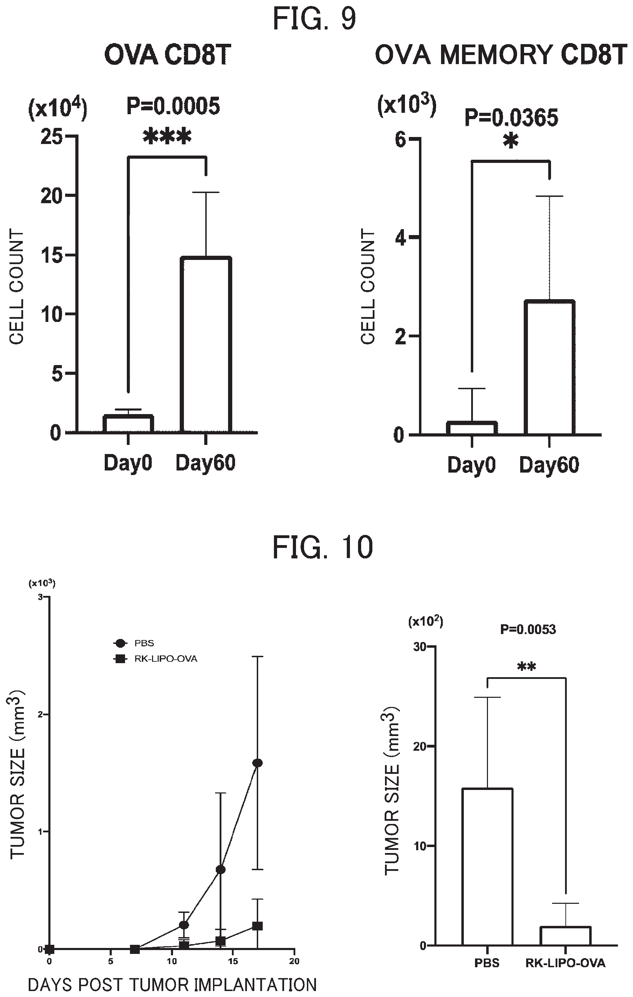

FIG. 1 shows the results of Test Example 1. In FIG. 1, RK-163-LIPO represents a group of mice administered with an RK-163-containing liposome composition, RCAI-61-LIPO a group of mice administered with an RCAI-61-containing liposome composition, RCAI-137-LIPO a group of mice administered with an RCAI-137-containing liposome composition, and PBS a group of mice administered with PBS (control). Part (A) of FIG. 1 shows PD-1 expression upon activation of CD8 T cells. Part (B) of FIG. 1 shows PD-1 expression upon activation of CD4 T cells. Part (C) of FIG. 1 shows PD-1 expression upon activation of NK cells. FIG. 2 shows the results of Test Example 2. In FIG. 2, RK-163-LIPO represents a group of mice administered with an RK-163-containing liposome composition, RCAI-61-LIPO a group of mice administered with an RCAI-61-containing liposome composition, RCAI-137-LIPO a group of mice administered with an RCAI-137-containing liposome composition, and PBS a group of mice administered with PBS (control). FIG. 3 shows the results of Test Example 3. In FIG. 3, RK-163-LIPO represents a group where an RK-163-containing liposome composition is used for mixed culture, RCAI-61-LIPO a group where an RCAI-61-containing liposome composition is used for mixed culture, RCAI-137-LIPO a group where an RCAI-137-containing liposome composition is used for mixed culture, and PBS a group where PBS is used instead of the liposome composition (control). FIG. 4 shows the results of Test Example 4. FIG. 5-1 shows the results of Test Example 5 (using an RK-163-containing liposome composition as the NKT cell ligand-containing liposome composition). In FIG. 5-1, PBS + ANTI-PD-1 represents a group of mice administered with an anti-PD-1 antibody alone, RK-163-LIPO a group of mice administered with an RK-163-containing liposome composition alone, and RK-163-LIPO + ANTI-PD-1 a group of mice administered with a combination of an RK-163-containing liposome composition and an anti-PD-1 antibody. FIG. 5-2 shows the results of Test Example 5 (using an RCAI-61-containing liposome composition as the NKT cell ligand-containing liposome composition). In FIG. 5-2, PBS + ANTI-PD-1 represents a group of mice administered with an anti-PD-1 antibody alone, RCAI-61-LIPO a group of mice administered with an RCAI-61-containing liposome composition alone, and RCAI-61-LIPO + ANTI-PD-1 a group of mice administered with a combination of an RCAI-61-containing liposome composition and an anti-PD-1 antibody. FIG. 5-3 shows the results of Test Example 5 (using an RCAI-137-containing liposome composition as the NKT cell ligand-containing liposome composition). In FIG. 5-3, PBS + ANTI-PD-1 represents a group of mice administered with an anti-PD-1 antibody alone, RCAI-137-LIPO a group of mice administered with an RCAI-137-containing liposome composition alone, and RCAI-137-LIPO + ANTI-PD-1 a group of mice administered with a combination of an RCAI-137-containing liposome composition and an anti-PD-1 antibody. FIG. 6 shows the results of Test Example 6. In FIG. 6, "0.1 ng" represents a group of mice administered with a liposome composition containing an NKT cell ligand (RK-163) and a surrogate cancer antigen (OVA) (RK-LIPO-OVA) in a dose of 0.1 ng (on RK-163 basis)/25 g body weight, "1 ng" a group of mice administered with RK-LIPO-OVA in a dose of 1 ng (on RK-163 basis)/25 g body weight, "10 ng" a group of mice administered with RK-LIPO-OVA in a dose of 10 ng (on RK-163 basis)/25 g body weight, and PBS a group of mice administered with PBS (control). The middle panel in FIG. 6 shows a bar graph plotting the tumor sizes over 17 days post tumor implantation, shown in the left panel. The data on the RK-LIPO-OVA dose (on RK-163 basis)-dependent tumor size shown in the middle panel were used to form the curve graph (right panel) and to calculate ED50. FIG. 7 shows the results of Test Example 7. In FIG. 7, RK-LIPO-OVA represents a group of mice administered with a liposome composition containing an NKT cell ligand (RK-163) and a surrogate cancer antigen (OVA), and PBS a group of mice administered with PBS (control). FIG. 8 shows the results of Test Example 8. In FIG. 8, RK-LIPO-OVA represents a group of mice administered with a liposome composition containing an NKT cell ligand (RK-163) and a surrogate cancer antigen (OVA), and PBS a group of mice administered with PBS (control). FIG. 9 shows the results of Test Example 9. FIG. 10 shows the results of Test Example 10. In FIG. 10, RK-LIPO-OVA represents a group of mice administered with a liposome composition containing an NKT cell ligand (RK-163) and a surrogate cancer antigen (OVA), and PBS a group of mice administered with PBS (control).

### PREFERRED MODE FOR CARRYING OUT THE INVENTION

### 1. First Mode of the Invention

Hereinafter, the first mode of the present invention will be described in detail. The present invention is directed to an antitumor agent including a combination of an immune checkpoint inhibitor and an NKT cell ligand-containing liposome composition (hereinafter also referred to as the "combination drug" of the present invention).

### [1. NKT Cell Ligand]

The NKT cell ligand-containing liposome composition of the present invention contains an NKT cell ligand. In the present invention, the NKT cell ligand is preferably a glycolipid selected from the group consisting of a compound represented by Formula (I) below, a compound represented by Formula (II) below, and salts of the foregoing compounds. The term "NKT cell ligand" refers to a compound that, when presented on the CDld molecule, can be specifically recognized by the NKT cell-specific antigen recognition receptor (NKT cell receptor) on NKT cells to specifically activate NKT cells. Herein, a compound represented by Formula (I), a compound represented by Formula (II), and salts of the foregoing compounds are also collectively referred to as "the NKT cell ligand of the present invention."

First, the compound represented by Formula (I) below (herein also referred to as "the compound (I)") and a salt thereof will be described. Formula (I): 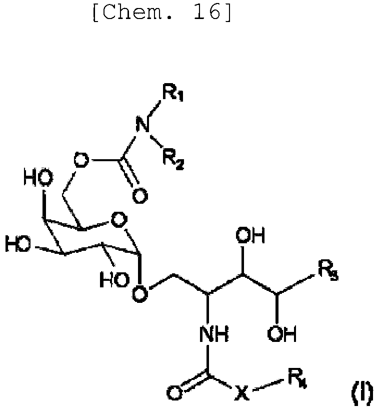 wherein X represents an alkylene group or -NH-,
R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with the adjacent nitrogen atom,
R₃ represents a hydrocarbon group having 1 to 20 carbon atoms, and
R₄ represents a hydrocarbon group having 1 to 30 carbon atoms.

X represents an alkylene group or -NH-. The alkylene group may be, for example, a linear or branched alkylene group having 1 to 8 carbon atoms. Specifically, the alkylene group may be methylene, ethylene, trimethylene, tetramethylene, pentamethylene, hexamethylene, heptamethylene, octamethylene, propylene, ethylethylene, dimethylmethylene, or dimethyltrimethylene.

R₁ and R₂ are the same or different and each represents a hydrogen atom, an alkyl group, a hydroxyl group, an alkoxy group, or an optionally-substituted aryl group, and R₁ and R₂ may form a 5- or 6-membered ring together with the adjacent nitrogen atom.

The alkyl group is typically linear or branched C₁₋₂₄ alkyl, more preferably linear or branched C₁₋₁₆ alkyl, even more preferably linear or branched C₁₋₁₀ alkyl, furthermore preferably linear or branched C₁₋₆ alkyl. Specifically, the alkyl group may be methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, or octadecyl. The alkyl group for R₁ or R₂ is preferably C₁₋₆ alkyl (e.g., methyl, ethyl).

The alkoxy group is typically linear or branched C₁₋₂₄ alkoxy, more preferably linear or branched C₁₋₁₆ alkoxy, even more preferably linear or branched C₁₋₁₀ alkoxy, furthermore preferably linear or branched C₁₋₆ alkoxy. Specifically, the alkoxy group may be methoxy, ethoxy, propoxy, isopropoxy, butoxy, or tert-butoxy. The alkoxy group for R₁ or R₂ is preferably C₁₋₆ alkoxy (e.g., methoxy).

Regarding the optionally-substituted aryl group, the aryl group is typically monocyclic, dicyclic, or tricyclic C₆₋₁₄ aryl, more preferably monocyclic, dicyclic, or tricyclic C₆₋₁₂ aryl. Specifically, the aryl group may be phenyl, naphthyl, anthryl, or phenanthryl. The aryl group for R₁ or R₂ is preferably C₆₋₁₂ aryl (e.g., phenyl). The aryl group may have a substituent(s). Examples of such a substituent(s) include halogen atoms (e.g., chlorine, fluorine, bromine, iodine); alkyl groups (e.g., methyl, ethyl, n-propyl, isopropyl, n-butyl, isobutyl, sec-butyl, tert-butyl, pentyl, hexyl); halogenoalkyl groups (e.g., trifluoromethyl); alkoxy groups (e.g., methoxy, ethoxy, propoxy, isopropoxy, butoxy, tert-butoxy); a hydroxyl group; an amino group; alkylamino groups (e.g., methylamino, dimethylamino, ethylamino, diethylamino); and cycloalkylamino groups. The aryl group may have any number of substituents at any positions. The number of substituents at any possible positions may be one to the possible maximum number.

R₁ and R₂ may form a 5- or 6-membered ring together with the adjacent nitrogen. Such a 5- or 6-membered ring is typically a 5- or 6-membered, nitrogen-containing saturated heterocyclic ring and specifically pyrrolidine, piperidine, morpholine, thiomorpholine, or piperazine. Such a 5- or 6-membered ring is preferably pyrrolidine, piperidine, or morpholine.

R₃ represents a hydrocarbon group having 1 to 20 carbon atoms. The term "hydrocarbon group having 1 to 20 carbon atoms" is intended to include C₁₋₂₀ alkyl groups, C₂₋₂₀ alkenyl groups, C₂₋₂₀ alkynyl groups, C₃₋₂₀ cycloalkyl groups, C₃₋₂₀ cycloalkenyl groups, and C₆₋₂₀ aryl groups. The hydrocarbon group having 1 to 20 carbon atoms may be linear, branched, or cyclic, may be saturated or unsaturated, and may have an intramolecular or terminal unsaturated bond. In particular, R₃ is preferably C₁₋₂₀ alkyl, C₂₋₂₀ alkenyl, or C₂₋₂₀ alkynyl, more preferably C₁₂₋₁₄ alkyl. Specifically, R₃ may be -(CH₂)₁₃CH₃.

R₄ represents a hydrocarbon group having 1 to 30 carbon atoms. The term "hydrocarbon group having 1 to 30 carbon atoms" is intended to include C₁₋₃₀ alkyl groups, C₂₋₃₀ alkenyl groups, C₂₋₃₀ alkynyl groups, C₃₋₃₀ cycloalkyl groups, C₃₋₃₀ cycloalkenyl groups, and C₆₋₃₀ aryl groups. The hydrocarbon group having 1 to 30 carbon atoms may be linear, branched, or cyclic, may be saturated or unsaturated, and may have an intramolecular or terminal unsaturated bond. In particular, R₄ is preferably C₁₋₃₀ alkyl, C₂₋₃₀ alkenyl, or C₂₋₃₀ alkynyl, more preferably C₁₀₋₃₀ alkyl, even more preferably C₁₅₋₂₅ alkyl. Specifically, R₄ may be -(CH₂)₁₅CH₃ or -(CH₂)₂₃CH₃.

The hydrocarbon group for R₃ or R₄ may have a substituent(s). The hydrocarbon group for R₃ or R₄ may have at least one substituent selected from the group consisting of halogen atoms (preferably chlorine or fluorine), alkoxy groups such as methoxy, ethoxy, propoxy, isopropoxy, butoxy, and tert-butoxy (preferably having 1 to 24 carbon atoms, more preferably 1 to 16 carbon atoms, even more preferably 1 to 10 carbon atoms, furthermore preferably 1 to 4 carbon atoms), aryloxy groups such as phenoxy (preferably having 6 to 14 carbon atoms), a hydroxyl group, an amino group, alkylamino groups such as methylamino, dimethylamino, ethylamino, and diethylamino, cycloalkylamino groups, alkylcarbonylamino groups such as acetamide, cycloalkylcarbonylamino groups, arylcarbonylamino groups such as benzoylamino (preferably arylcarbonylamino groups having a C₆₋₁₄ aryl moiety), and other electron-donating groups; a carboxyl group, alkoxycarbonyl groups, and an acyl group (as described later). The substituent is preferably an alkyl-carbonyl group having a linear or branched C₁₋₂₄ alkyl moiety, a carbamoyl group, or an electron-withdrawing group, such as trifluoromethyl. The hydrocarbon group may have any number of substituents at any positions. The number of substituents at any possible positions may be one to the possible maximum number. The hydrocarbon group may have one or more substituents, which may be the same or different.

For example, the acyl group may be a formyl group, an alkyl-carbonyl group (e.g., an alkyl-carbonyl group having a linear or branched C₁₋₂₄ (preferably C₁₋₁₂) alkyl moiety, such as acetyl, propionyl, butyryl, isobutyryl, valeryl, pivaloyl, or hexanoyl), a cycloalkyl-carbonyl group (e.g., a cycloalkyl-carbonyl group having a C₃₋₁₀ cycloalkyl moiety), an alkenyl-carbonyl group (e.g., an alkenyl-carbonyl group having a linear or branched C₂₋₁₂ alkenyl moiety, such as acryloyl or methacryloyl), or an aryl-carbonyl group (e.g., an aryl-carbonyl group having a C₆₋₁₄ aryl moiety, such as benzoyl or naphthoyl). For example, the aryl group of the aryl-carbonyl group may be a monocyclic, dicyclic, or tricyclic aromatic hydrocarbon group, such as phenyl, naphthyl, anthryl, or phenanthryl. In particular, the acyl group is preferably formyl, acetyl, propionyl, butyryl, isobutyryl, benzoyl, or naphthoyl, more preferably acetyl or benzoyl.

The alkyl moiety of the alkylamino or alkylcarbonylamino group may be methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, octadecyl, or any other linear or branched alkyl group (preferably having 1 to 24 carbon atoms, more preferably 1 to 16 carbon atoms, even more preferably 1 to 10 carbon atoms, furthermore preferably 1 to 4 carbon atoms). The cycloalkyl moiety of the cycloalkylamino or cycloalkylcarbonylamino group may be cyclopentyl, cyclohexyl, or any other cycloalkyl group (preferably having 3 to 24 carbon atoms, more preferably 3 to 16 carbon atoms, even more preferably 3 to 10 carbon atoms, furthermore preferably 3 to 6 carbon atoms). The alkoxy moiety of the alkoxycarbonyl group may be the same as that shown above.

The substituent may be further substituted with a substituent at any possible position. Such a substituent may be at least one selected from the group consisting of halogen, alkyl, cycloalkyl, alkenyl, alkynyl, phenyl, alkoxy, hydroxyl, amino, alkylamino, and cycloalkylamino. Examples of halogen, alkoxy, alkylamino, and cycloalkylamino include those listed above. The alkyl group may be methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, octadecyl, or any other alkyl group (preferably having 1 to 24 carbon atoms, more preferably 1 to 16 carbon atoms, even more preferably 1 to 10 carbon atoms, furthermore preferably 1 to 4 carbon atoms). The cycloalkyl group may be cyclopentyl, cyclohexyl, or any other cycloalkyl group (preferably having 3 to 24 carbon atoms, more preferably 3 to 16 carbon atoms, even more preferably 3 to 10 carbon atoms, furthermore preferably 3 to 6 carbon atoms). The alkenyl group may be vinyl, propenyl, butenyl, or any other alkenyl group (preferably having 2 to 24 carbon atoms, more preferably 2 to 16 carbon atoms, even more preferably 2 to 10 carbon atoms, furthermore preferably 2 to 4 carbon atoms). The alkynyl group may be ethynyl, propargyl, butynyl, pentynyl, or any other alkynyl group (preferably having 2 to 24 carbon atoms, more preferably 2 to 16 carbon atoms, even more preferably 2 to 10 carbon atoms, furthermore preferably 2 to 4 carbon atoms).

In the present invention, the stereoisomeric form of the sugar moiety (galactopyranose) having a cyclic structure should be the α-anomer. The compound (I) may also have a stereoisomeric form with respect to a structure other than the cyclic structure of the sugar moiety (e.g., with respect to any asymmetric carbon atom in a moiety other than the cyclic structure of the sugar moiety). The present invention encompasses any type of such isomers, and the compound (I) may be a mixture (including a racemic mixture) of two or more isomers in any ratio. Specifically, the compound (I) may be in the form of an optical isomer with respect to an asymmetric carbon atom in a moiety other than the cyclic structure of the sugar moiety. In the present invention, the compound (I) may also be a single optically-active substance or a mixture (including a racemic mixture) of two or more optically-active substances in any ratio. The asymmetric carbon atom to which - NHC(=O)X-R₄ is bonded preferably has an S-configuration. The asymmetric carbon atom to which OH is bonded adjacent to the - NHC(=O)X-R₄-bonded asymmetric carbon atom preferably has an R-configuration. The asymmetric carbon atom to which R₃ is bonded preferably has an R-configuration.

The salt of the compound (I) is preferably a pharmaceutically acceptable salt. For example, the salt of the compound (I) may be hydrochloride, hydrobromide, hydroiodide, sulfate, nitrate, phosphate, or any other inorganic acid salt; succinate, fumarate, acetate, methanesulfonate, toluenesulfonate, or any other organic acid salt; sodium salt, potassium salt, or any other alkali metal salt; magnesium salt, calcium salt, or any other alkaline-earth metal salt; or ammonium salt, alkylammonium salt, or any other ammonium salt.

Preferred examples of the compound (I) or the salt thereof include the following:
(a) a compound of Formula (I) wherein
   X is -CH₂-,
   R₁ and R₂ are the same or different and are each a hydrogen atom, a C₁₋₆ alkyl group, a hydroxyl group, or a C₁₋₆ alkoxy group,
   R₃ is a C₁₋₂₀ alkyl group, and
   R₄ is a C₁₋₃₀ alkyl group, or a salt thereof;
(b) a compound of Formula (I) wherein
   X is -CH₂-,
   R₁ and R₂ are the same or different and are each a hydrogen atom, a C₁₋₆ alkyl group, a hydroxyl group, or a C₁₋₆ alkoxy group,
   R₃ is a C₁₂₋₁₄ alkyl group, and
   R₄ is a C₁₅₋₂₅ alkyl group, or a salt thereof;
(c) a compound of Formula (I) wherein
   X is -CH₂-,
   R₁ and R₂ are the same or different and are each a hydrogen atom, a C₁₋₆ alkyl group, or a hydroxyl group,
   R₃ is a C₁₂₋₁₄ alkyl group, and
   R₄ is a C₁₅₋₂₅ alkyl group, or a salt thereof; and
(d) a compound of Formula (I) wherein
   X is -CH₂-,
   R₁ and R₂ are the same or different and are each a hydrogen atom, methyl, or hydroxyl,
   R₃ is -(CH₂)₁₃CH₃, and
   R₄ is -(CH₂)₂₃CH₃, or a salt thereof.

In the present invention, preferred examples of the compound (I) include, but are not limited to, those shown in Table 1.

**[Table 1]**

| | 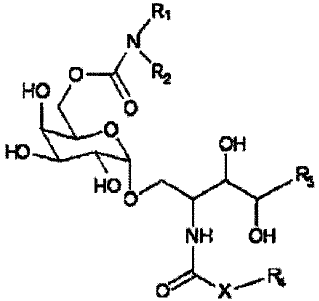 | | | |
|---|---|---|---|---|
| | -NR₁R₂ | -X- | -R₃ | -R₄ |
| RCAI-123 | -NMe₂ | -CH₂- | -C₁₄H₂₉ | -C₂₄H₄₉ |
| RCAI-124 | -NHMe | -CH₂- | -C₁₄H₂₉ | -C₂₄H₄₉ |
| RCAI-137 | -NHOH | -CH₂- | -C₁₄H₂₉ | -C₂₄H₄₉ |
| RCAI-138 | -NHOMe | -CH₂- | -C₁₄H₂₉ | -C₂₄H₄₉ |
| RCAI-148 | -NHEt | -CH₂- | -C₁₄H₂₉ | -C₂₄H₄₉ |
| RCAI-149 | -NEt₂ | -CH₂- | -C₁₄H₂₉ | -C₂₄H₄₉ |
| RCAI-121 | 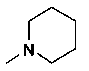 | -CH₂- | -C₁₄H₂₉ | -C₂₄H₄₉ |
| RCAI-122 | 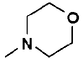 | -CH₂- | -C₁₄H₂₉ | -C₂₄H₄₉ |
| RCAI-131 | 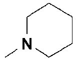 | -CH₂- | -C₁₄H₂₉ | -C₂₄H₄₉ |
| RCAI-132 | 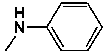 | -CH₂- | -C₁₄H₂₉ | -C₂₄H₄₉ |
| RCAI-139 | 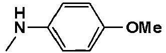 | -CH₂- | -C₁₄H₂₉ | -C₂₄H₄₉ |
| RCAI-140 | 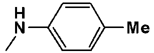 | -CH₂- | -C₁₄H₂₉ | -C₂₄H₄₉ |
| RCAI-141 | 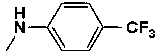 | -CH₂- | -C₁₄H₂₉ | -C₂₄H₄₉ |
| RCAI-150 | -NMe₂ | -NH- | -C₁₄H₂₉ | -C₁₆H₃₃ |

| | | | | |
|---|---|---|---|---|
| In the table, -C14H29 represents -(CH2)13CH3, -C24H49 represents -(CH2)23CH3, and -C16H33 represents -(CH2)15CH3. | | | | |

In particular, the compound (I) is preferably the following compound:
RCAI-124 (herein also referred to as RK-163) below; or

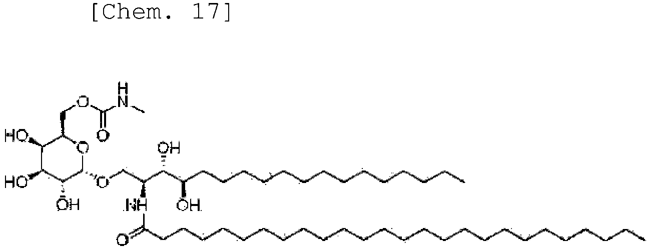

RCAI-137 below.

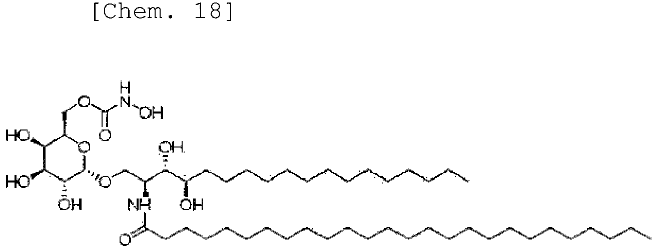

The compound represented by Formula (I) or a salt thereof can be produced using methods disclosed in publications such as WO2013/162016A1 and US2015/152128A1.

Next, the compound represented by Formula (II) below (herein also referred to as the compound (II)) and the salt thereof will be described. Formula (II): 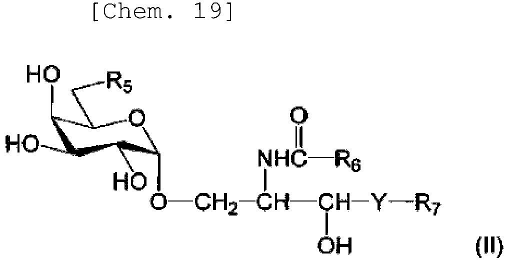 wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.

R₅ represents a hydrogen atom, an alkyl group having 1 to 7 carbon atoms, an alkoxy group having 1 to 6 carbon atoms, or a halogen atom. The alkyl group for R₅ having 1 to 7 carbon atoms may be a substituted, unsubstituted, or cyclic alkyl group. The alkyl group for R₅ is typically methyl, ethyl, n-propyl, isopropyl, cyclopropyl, cyclopropylmethyl, n-butyl, isobutyl, sec-butyl, tert-butyl, pentyl, hexyl, heptyl, or cyclohexylmethyl, preferably methyl or ethyl. The alkoxy group for R₅ having 1 to 6 carbon atoms may be an alkyl-O-group having a substituted or unsubstituted alkyl moiety bonded to the oxygen atom, in which the alkyl moiety may be cyclic. The alkoxy group for R₅ is typically methoxy, ethoxy, n-propyloxy, isopropyloxy, cyclopropyloxy, cyclopropylmethyloxy, n-butoxy, isobutyloxy, sec-butyloxy, tert-butyloxy, pentyloxy, hexyloxy, or cyclohexyloxy, preferably methoxy, ethoxy, or n-propyloxy. The halogen atom for R₅ is typically fluorine, chlorine, bromine, or iodine, preferably fluorine or chlorine.

R₆ and R₇ each independently represent a substituted or unsubstituted hydrocarbon group having 1 to 28 carbon atoms. Herein, the term "substituted or unsubstituted hydrocarbon group having 1 to 28 carbon atoms" is intended to include substituted or unsubstituted C₁₋₂₈ alkyl groups, substituted or unsubstituted C₂₋₂₈ alkenyl groups, substituted or unsubstituted C₂₋₂₈ alkynyl groups, substituted or unsubstituted C₃₋₂₈ cycloalkyl groups, substituted or unsubstituted C₃₋₂₈ cycloalkenyl groups, substituted or unsubstituted C₆₋₁₄ aryl groups, and substituted or unsubstituted C₇₋₂₈ arylalkyl groups (e.g., C₆₋₁₄ aryl-C₁₋₁₄ alkyl groups). The substituted or unsubstituted hydrocarbon group having 1 to 28 carbon atoms may be linear, branched, or cyclic, may be saturated or unsaturated, and may have an intramolecular or terminal unsaturated bond. In particular, R₆ or R₇ is preferably a substituted or unsubstituted alkyl group having 1 to 28 carbon atoms or a substituted or unsubstituted arylalkyl group having 7 to 28 carbon atoms. When R₅ is a hydrogen atom, R₆ should preferably be a substituted or unsubstituted hydrocarbon group having 24 to 28 carbon atoms, more preferably a substituted or unsubstituted alkyl group having 24 to 28 carbon atoms.

The hydrocarbon group for R₆ or R₇ may have at least one substituent selected from the group consisting of halogen atoms (preferably chlorine or fluorine), alkoxy groups such as methoxy, ethoxy, propoxy, isopropoxy, butoxy, and tert-butoxy (preferably having 1 to 24 carbon atoms, more preferably 1 to 16 carbon atoms, even more preferably 1 to 10 carbon atoms, furthermore preferably 1 to 4 carbon atoms), aryloxy groups such as phenoxy (preferably having 6 to 14 carbon atoms), a hydroxyl group, an amino group, alkylamino groups such as methylamino, dimethylamino, ethylamino, and diethylamino, cycloalkylamino groups, alkylcarbonylamino groups such as acetamide, cycloalkylcarbonylamino groups, arylcarbonylamino groups such as benzoylamino (preferably arylcarbonylamino groups having a C₆₋₁₄ aryl moiety), and other electron-donating groups; a carboxyl group, alkoxycarbonyl groups, and an acyl group (as described later). The substituent is preferably an alkyl-carbonyl group having a linear or branched C₁₋₂₄ alkyl moiety, a carbamoyl group, or an electron-withdrawing group, such as trifluoromethyl.

For example, the acyl group may be a formyl group, an alkyl-carbonyl group (e.g., an alkyl-carbonyl group having a linear or branched C₁₋₂₄ (preferably C₁₋₁₂) alkyl moiety, such as acetyl, propionyl, butyryl, isobutyryl, valeryl, pivaloyl, or hexanoyl), a cycloalkyl-carbonyl group (e.g., a cycloalkyl-carbonyl group having a C₃₋₁₀ cycloalkyl moiety), an alkenyl-carbonyl group (e.g., an alkenyl-carbonyl group having a linear or branched C₂₋₁₂ alkenyl moiety, such as acryloyl or methacryloyl), or an aryl-carbonyl group (e.g., an aryl-carbonyl group having a C₆₋₁₄ aryl moiety, such as benzoyl or naphthoyl). For example, the aryl group of the aryl-carbonyl group may be a monocyclic, dicyclic, or tricyclic aromatic hydrocarbon group, such as phenyl, naphthyl, anthryl, or phenanthryl. In particular, the acyl group is preferably formyl, acetyl, propionyl, butyryl, isobutyryl, benzoyl, or naphthoyl, more preferably acetyl or benzoyl.

The alkyl moiety of the alkylamino or alkylcarbonylamino group may be methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, octadecyl, or any other linear or branched alkyl group (preferably having 1 to 24 carbon atoms, more preferably 1 to 16 carbon atoms, even more preferably 1 to 10 carbon atoms, furthermore preferably 1 to 4 carbon atoms). The cycloalkyl moiety of the cycloalkylamino or cycloalkylcarbonylamino group may be cyclopentyl, cyclohexyl, or any other cycloalkyl group (preferably having 3 to 24 carbon atoms, more preferably 3 to 16 carbon atoms, even more preferably 3 to 10 carbon atoms, furthermore preferably 3 to 6 carbon atoms). The alkoxy moiety of the alkoxycarbonyl group may be the same as that shown above.

The substituent may be further substituted with a substituent at any possible position. Such a substituent may be at least one selected from the group consisting of halogen, alkyl, cycloalkyl, alkenyl, alkynyl, phenyl, alkoxy, hydroxyl, amino, alkylamino, and cycloalkylamino. Examples of halogen, alkoxy, alkylamino, and cycloalkylamino include those listed above. The alkyl group may be methyl, ethyl, n-propyl, isopropyl, n-butyl, isobutyl, sec-butyl, tert-butyl, pentyl, hexyl, heptyl, octyl, nonyl, decyl, undecyl, dodecyl, tridecyl, tetradecyl, pentadecyl, hexadecyl, heptadecyl, octadecyl, or any other alkyl group (preferably having 1 to 24 carbon atoms, more preferably 1 to 16 carbon atoms, even more preferably 1 to 10 carbon atoms, furthermore preferably 1 to 4 carbon atoms). The cycloalkyl group may be cyclopentyl, cyclohexyl, or any other cycloalkyl group (preferably having 3 to 24 carbon atoms, more preferably 3 to 16 carbon atoms, even more preferably 3 to 10 carbon atoms, furthermore preferably 3 to 6 carbon atoms). The alkenyl group may be vinyl, propenyl, butenyl, or any other alkenyl group (preferably having 2 to 24 carbon atoms, more preferably 2 to 16 carbon atoms, even more preferably 2 to 10 carbon atoms, furthermore preferably 2 to 4 carbon atoms). The alkynyl group may be ethynyl, propargyl, butynyl, pentynyl, or any other alkynyl group (preferably having 2 to 24 carbon atoms, more preferably 2 to 16 carbon atoms, even more preferably 2 to 10 carbon atoms, furthermore preferably 2 to 4 carbon atoms).

In particular, R₆ is preferably a substituted or unsubstituted alkyl group or a linear alkyl group. R₆ is also preferably a substituted or unsubstituted arylalkyl group. When R₅ is an alkyl group having 1 to 7 carbon atoms, an alkoxy group having 1 to 6 carbon atoms, or a halogen atom, the R₆ group preferably has 18 to 26 carbon atoms, more preferably 24 to 26 carbon atoms. When R₅ is a hydrogen atom, the R₆ group preferably has 24 to 26 carbon atoms. Specifically, R₆ may be -(CH₂)₂₃-CH₃, -(CH₂)₂₄-CH₃, -(CH₂)₂₅-CH₃, or 10-(4-fluorophenyl)decyl. R₇ is preferably a substituted or unsubstituted alkyl group or a linear alkyl group. The R₇ group preferably has 9 to 20 carbon atoms, more preferably 12 to 18 carbon atoms. Specifically, R₇ may be -(CH₂)₁₁-CH₃, - (CH₂)₁₂-CH₃, -(CH₂)₁₃-CH₃, -(CH₂)₁₄-CH₃, -(CH₂)₁₅-CH₃, -(CH₂)₁₆-CH₃, or -(CH₂)₁₇-CH₃.

Y represents -CH₂-, -CH(OH)-, or -CH=CH-. In particular, Y is preferably -CH(OH)-.

The compound (II) may have any stereoisomeric form. In such a case, the present invention encompasses any type of isomers, and the compound (II) may be a mixture (including a racemic mixture) of two or more isomers in any ratio. Specifically, the compound (II) may be in the form of an optical isomer with respect to the asymmetric carbon atom in the lipid moiety. In the present invention, the compound (II) may also be a single optically-active substance or a mixture (including a racemic mixture) of two or more optically-active substances in any ratio. The asymmetric carbon atom to which - NHCOR₆ is bonded preferably has an S-configuration. The asymmetric carbon atom to which -OH is bonded adjacent to the -NHCOR₆-bonded asymmetric carbon atom preferably has an anti-configuration with respect to the asymmetric carbon atom to which -NHCOR₆ is bonded. When Y is -CH(OH)-, the asymmetric carbon atom in the -CH(OH)- moiety represented by Y preferably has an R-configuration.

The lipid moiety of the compound (II) may be represented by the formula: 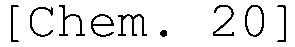 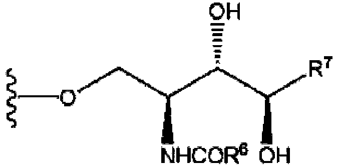 wherein each of the symbols is as defined above.

The salt of the compound (II) is preferably a pharmacologically acceptable salt. For example, the salt of the compound (II) may be hydrochloride, hydrobromide, hydroiodide, sulfate, nitrate, phosphate, or any other inorganic acid salt; succinate, fumarate, acetate, methanesulfonate, toluenesulfonate, or any other organic acid salt; sodium salt, potassium salt, or any other alkali metal salt; magnesium salt, calcium salt, or any other alkaline-earth metal salt; or ammonium salt, alkylammonium salt, or any other ammonium salt.

In the present invention, preferred examples of the compound (II) include, but are not limited to, those shown in Table 2.

**[Table 2]**

| Compound No. | 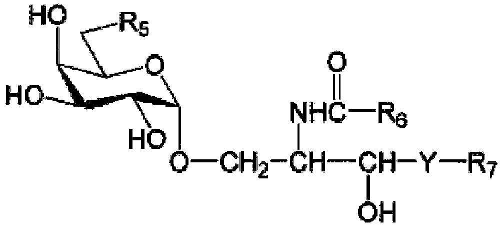 | | | |
|---|---|---|---|---|
| | R⁵ | R⁶ | Y | R⁷ |
| 2-9' | -OCH₃ | -(CH₂)₂₄CH₃ | 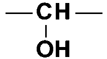 | -(CH₂)₁₃CH₃ |
| 3-4' | -CH₃ | -(CH₂)₂₄CH₃ | 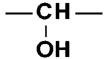 | -(CH₂)₁₃CH₃ |
| 3-7' | -F | -(CH₂)₂₄CH₃ | 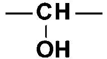 | -(CH₂)₁₃CH₃ |
| 3-10' | -H | -(CH₂)₂₄CH₃ | 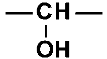 | -(CH₂)₁₃CH₃ |
| 3-13' | -OC₂H₅ | -(CH₂)₂₄CH₃ | 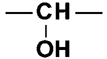 | -(CH₂)₁₃CH₃ |
| 3-16' | -O(CH)₂CH₃ | -(CH₂)₂₄CH₃ | 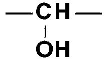 | -(CH₂)₁₃CH₃ |
| 165' | -OCH₃ | 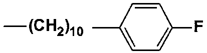 | 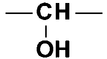 | -(CH₂)₁₃CH₃ |

In particular, the compound (II) is preferably at least one selected from the group consisting of:
Compound No. 2-9' (RCAI-61) shown in Table 2 and having the following steric structure:

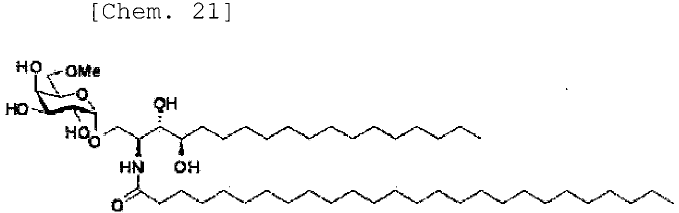

Compound No. 3-4' (RCAI-64) shown in Table 2 and having the following steric structure:

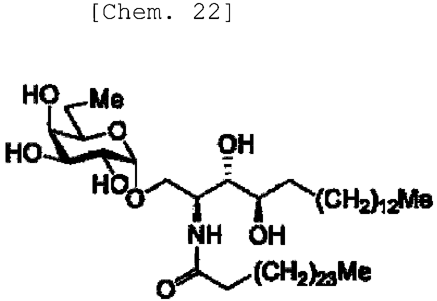

Compound No. 3-13' (RCAI-85) shown in Table 2 and having the following steric structure:

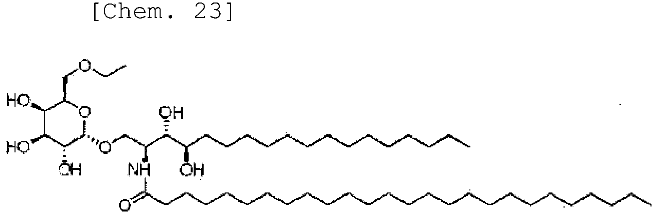

Compound No. 165' (RCAI-165) shown in Table 2 and having the following steric structure:

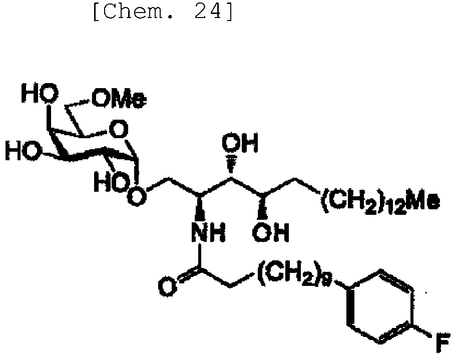

The compound represented by Formula (II) or the salt thereof can be produced using methods disclosed in publications such as WO2009/119692A1 and U. S. Patent No. 8,551,959.

### [2. Liposome]

The liposome composition of the present invention includes liposomes containing the glycolipid described above.

Herein, any material having a closed vesicle structure composed mainly of a lipid or any other suitable component (e.g., a water-soluble particle formed through aggregation of amphiphilic molecules having hydrophilic and hydrophobic regions) is referred to as a "liposome". Nowadays, a liposome may be called a lipid nanoparticle (LNP). As used herein, the term "liposome" is intended to also include any material called a "lipid nanoparticle" as long as it has such a structure. In the present invention, the liposome may include, as a component, any amphiphilic molecule that is capable of forming a lipid closed vesicle structure when subjected to a known process. Such a component is preferably a lipid. In the present invention, examples of the lipid include phosphatidylcholine (e.g., dioleoyl phosphatidylcholine, dilauroyl phosphatidylcholine, dimyristoyl phosphatidylcholine, dipalmitoyl phosphatidylcholine, distearoyl phosphatidylcholine), phosphatidylglycerol (e.g., dioleoyl phosphatidylglycerol, dilauroyl phosphatidylglycerol, dimyristoyl phosphatidylglycerol, dipalmitoyl phosphatidylglycerol, distearoyl phosphatidylglycerol), phosphatidylethanolamine (e.g., dioleoyl phosphatidylethanolamine, dilauroyl phosphatidylethanolamine, dimyristoyl phosphatidylethanolamine, dipalmitoyl phosphatidylethanolamine, distearoyl phosphatidylethanolamine), phosphatidylserine, phosphatidylinositol, phosphatidic acid, cardiolipin, and other phospholipids; sphingoglycolipids, glyceroglycolipids, cationic lipids (e.g., DOTAP, DLin-MC3-DMA ((6Z,9Z,28Z,31Z)-heptatriaconta-6,9,28,31-tetraen-19-yl-4-(dimethylamino)butanoate), ALC-0315 ([(4-hydroxybutyl)azanediyl]bis(hexane-6,1-diyl)bis(2-hexyldecanoate)), and SM-102 (heptadecan-9-yl-8-((2-hydroxyethyl) (6-oxo-6-(undecyloxy)hexyl)amino)octanoate). One or more of these lipids may be used to form the liposome, or one or more of these lipids may be used in combination with a lipid derivative having a lipid moiety bonded to a nonpolar moiety, such as a cholesterol moiety, or a water-soluble polymer moiety, such as a polyethylene glycol moiety, to form the liposome.

In the present invention, the liposome preferably has affinity for antigen-presenting cells. In the present invention, preferred examples of the liposome (e.g., the liposome having affinity for antigen-presenting cells) include Liposomes #2 to #8 shown below.

### Liposome #2: A liposome including DOTAP, DOPE, and cholesterol

In Liposome #2, the weight ratio of DOTAP, DOPE, and cholesterol (DOTAP:DOPE:cholesterol) is, typically 1:0.1-10:0.1-10, preferably 1:0.3-3:0.2-2. The liposome composition of the present invention may include Liposome #2 and the NKT cell ligand according to the present invention, in which the ratio of the weight of the NKT cell ligand to the total weight of DOTAP, DOPE, and cholesterol is typically 1:1-10,000, preferably 1:1-1,000.

### Liposome #3: A liposome including DOTAP, DOPE, MPEG2000-DMPE Na salt, and cholesterol

In Liposome #3, the weight ratio of DOTAP, DOPE, MPEG2000-DMPE Na salt, and cholesterol (DOTAP:DOPE:MPEG2000-DMPE Na salt:cholesterol) is typically 1:0.1-10:0.1-10:0.1-10, preferably 1:0.3-3:0.2-2:0.2-2. The liposome composition of the present invention may include Liposome #3 and the NKT cell ligand according to the present invention, in which the ratio of the weight of the NKT cell ligand to the total weight of DOTAP, DOPE, MPEG2000-DMPE Na salt, and cholesterol is typically 1:1-10,000, preferably 1:1-1,000.

### Liposome #4: A liposome including DOTAP, DOPE, MPEG2000-DSPE Na salt, and cholesterol

In Liposome #4, the weight ratio of DOTAP, DOPE, MPEG2000-DSPE Na salt, and cholesterol (DOTAP:DOPE:MPEG2000-DSPE Na salt:cholesterol) is typically 1:0.1-10:0.1-10:0.1-10, preferably 1:0.3-3:0.2-2:0.2-2. The liposome composition of the present invention may include Liposome #4 and the NKT cell ligand according to the present invention, in which the ratio of the weight of the NKT cell ligand to the total weight of DOTAP, DOPE, MPEG2000-DSPE Na salt, and cholesterol is typically 1:1-10,000, preferably 1:1-1,000.

### Liposome #5: A liposome including HSPC, DSPG, and cholesterol

In Liposome #5, the weight ratio of HSPC, DSPG, and cholesterol (HSPC:DSPG:cholesterol) is typically 1:0.1-10:0.1-10, preferably 1:0.2-2:0.2-2. The liposome composition of the present invention may include Liposome #5 and the NKT cell ligand according to the present invention, in which the ratio of the weight of the NKT cell ligand to the total weight of HSPC, DSPG, and cholesterol is typically 1:1-10,000, preferably 1:1-1,000.

### Liposome #6: A liposome including HSPC, DOPS, and cholesterol

Liposome #6 and Liposome #7 shown below share the same components. For example, based on the amount of the NKT cell ligand, the former may be used in a relatively large amount to form the NKT cell ligand-containing liposome, while the latter may be used in a relatively small amount to form the NKT cell ligand-containing liposome. In Liposome #6, the weight ratio of HSPC, DOPS, and cholesterol (HSPC:DOPS:cholesterol) is typically 1:0.1-10:0.1-10, preferably 1:0.2-2:0.2-2. The liposome composition of the present invention may include Liposome #6 and the NKT cell ligand according to the present invention, in which the ratio of the weight of the NKT cell ligand to the total weight of HSPC, DOPS, and cholesterol is typically 1:1-100,000, preferably 1:1-10,000.

### Liposome #7: A liposome including HSPC, DOPS, and cholesterol

In Liposome #7, the weight ratio of HSPC, DOPS, and cholesterol (HSPC:DOPS:cholesterol) is typically 1:0.1-10:0.1-10, preferably 1:0.2-2:0.2-2. The liposome composition of the present invention may include Liposome #7 and the NKT cell ligand according to the present invention, in which the ratio of the weight of the NKT cell ligand to the total weight of HSPC, DOPS, and cholesterol is typically 1:1-10,000, preferably 1:1-1,000. The liposome composition containing Liposome #7 according to an embodiment of the present invention may be, for example, an RK-163-containing liposome composition, an RCAI-61-containing liposome composition, or an RCAI-137-containing liposome composition as shown in Examples 1 and 2 below.

### Liposome #8: A liposome including HSPC, MPEG2000-DSPE Na salt, and cholesterol

In Liposome #8, the weight ratio of HSPC, MPEG2000-DSPE Na salt, and cholesterol (HSPC:MPEG2000-DSPE Na salt:cholesterol) is typically 1:0.1-10:0.1-10, preferably 1:0.2-2:0.2-2. The liposome composition of the present invention may include Liposome #8 and the NKT cell ligand according to the present invention, in which the ratio of the NKT cell ligand to the total weight of HSPC, MPEG2000-DSPE Na salt, and cholesterol is typically 1:1-10,000, preferably 1:1-1,000.

In the present invention, the liposome is preferably any one of Liposomes #5 to #8, more preferably Liposome #7 or #8, even more preferably Liposome #7.

### [3. NKT Cell Ligand-Containing Liposome Composition]

In the present invention, the NKT cell ligand-containing liposome composition includes liposomes, which may be prepared using known methods. For example, such methods are described in Liposome Technology, Vol. 1, 2nd Edition (by Gregory Gregoriadis (CRC Press, Boca Raton, Ann Arbor, London, Tokyo), Chapter 4, pp.67-80, Chapter 10, pp.167-184, and Chapter 17, pp.261-276 (1993). More specifically, such methods include, but are not limited to, ultrasonication, ethanol injection, French press, ether injection, cholate method, calcium-induced fusion, freeze-thaw, and reverse-phase evaporation. Specifically, as shown in the section "Examples", liposome components and the NKT cell ligand may be dissolved in an organic solvent in a glass test tube. Nitrogen gas may be blown to the solution so that the organic solvent can be removed by evaporation. The resulting residue may be completely dried in a desiccator (under reduced pressure). The dried product may be mixed with an isotonic aqueous solution (e.g., 9% sucrose aqueous solution, 5% glucose aqueous solution, phosphate-buffered saline), and the mixture may be emulsified using a homogenizer to form NKT cell ligand-containing liposomes. The liposomes may further be passed through a polycarbonate membrane filter (with a pore size of 0.4 µm or 0.2 µm) at increased temperature and pressure to give uniform-sized liposomes. In general, finally, the liposomes may also be passed through a sterilizing filter (e.g., PVDF membrane filter with a pore size of 0.22 µm) for reliable sterility.

In the present invention, the NKT cell ligand-containing liposome composition is preferably an RK-163-containing liposome composition, an RCAI-61-containing liposome composition, or an RCAI-137-containing liposome composition. The NKT cell ligand-containing composition is more preferably an RK-163-containing liposome composition, an RCAI-61-containing liposome composition, or an RCAI-137-containing liposome composition, in each of which the liposome is Liposome #7 shown above.

In the NKT cell ligand-containing liposome composition of the present invention, the ratio of the weight of the glycolipid selected from the group consisting of the compound of Formula (I), the compound of Formula (II), and salts of the foregoing compounds (the NKT cell ligand according to the present invention) to the total weight of other lipids (components other than the NKT cell ligand (main component)) constituting the liposomes is typically 1:1-100,000, preferably 1:1-10,000, more preferably 1:1-1,000, even more preferably 1:1-100.

As a non-limiting example, the NKT cell ligand-containing liposome composition of the present invention typically has an average particle size of 10 to 1,000 nm, preferably 10 to 500 nm, more preferably 50 to 500 nm. The average particle size of the liposomes can be measured using conventional quasi-elastic light scattering.

In the present invention, the NKT cell ligand-containing liposome composition may be formulated alone into a preparation, or the NKT cell ligand-containing liposome composition may be formulated together with a conventional pharmaceutically-acceptable additive (e.g., solvent, stabilizer, isotonic agent, analgesic, buffer, pH adjuster) into a preparation. The liposome composition of the present invention may be a liquid preparation, such as an injection. In such a case, the liquid preparation may be cryopreserved or may be stored after being subjected to water removal by freeze drying. The freeze-dried preparation may be mixed with distilled water for injection and dissolved again for use.

### [4. Immune Checkpoint Inhibitor]

In the present invention, the immune checkpoint inhibitor is an agent that inhibits the function of an immune checkpoint molecule or an immune checkpoint molecule ligand. Examples of the immune checkpoint inhibitor include, but are not limited to, anti-PD-1 antibodies and anti-PD-L1 antibodies (anti-PD-1 ligand antibodies). The immune checkpoint inhibitor may be a commercially available anti-PD-1 antibody, such as nivolumab (trade name: Opdivo) or pembrolizumab (trade name: Keytruda). Nivolumab is a recombinant human IgG4 monoclonal antibody against human PD-1, which has a substitution of Pro at residue 221 in the heavy chain. Nivolumab is produced in Chinese hamster ovary cells. Nivolumab is a glycoprotein composed of two heavy chains consisting of 440 amino acid residues and two light chains consisting of 214 amino acid residues (see package insert "Opdivo^{®} Intravenous Infusion 20 mg" revised June 2023). The immune checkpoint inhibitor may be a commercially available anti-PD-L1 antibody, such as atezolizumab (trade name: Tecentriq), avelumab (trade name: Bavencio), or durvalumab (trade name: Imfinzi). Tecentriq is a protein composed of two L chains consisting of 214 amino acid residues and two H chains consisting of 448 amino acid residues (see package insert "Tecentriq^{®} Intravenous Infusion 1200 mg" revised May 2022).

### [5. Combination of Immune Checkpoint Inhibitor and NKT Cell Ligand-Containing Liposome Composition]

The present invention is directed to administering the immune checkpoint inhibitor in combination with the NKT cell ligand-containing liposome, which is expected to produce synergistic antitumor effects.

Examples of tumors (malignant tumors) for which the combination of the immune checkpoint inhibitor and the NKT cell ligand-containing liposome composition is expected to be therapeutically effective include, but are not limited to, breast cancer, colorectal cancer, lung cancer, prostate cancer, esophageal cancer, gastric cancer, liver cancer, biliary tract cancer, spleen cancer, kidney cancer, bladder cancer, uterine cancer, testicular cancer, thyroid cancer, pancreatic cancer, brain tumors, ovarian cancer, skin cancer, melanoma, and hematologic tumors. The agent according to the present invention is also expected to be effective for subjects suffering from multiple cancers at the same time.

Examples of methods for administering the combination include, but are not limited to, intravenous administration, arterial administration, intramucosal administration, intranodal administration, and administration into affected tissues. The immune checkpoint inhibitor and the NKT cell ligand-containing liposome composition may be administered simultaneously or separately at different times. They may also be administered via different routes.

The NKT cell ligand of the NKT cell ligand-containing liposome composition may be administered in a dose sufficient to activate NKT cells in vivo and to treat the target disease. As a non-limiting example, the NKT cell ligand-containing liposome composition is typically administered in a dose of 400 ng/kg to 4 mg/kg. The administration may be performed multiple times depending on the condition of the subject or the severity of the disease. In experiments, an anti-PD-1 antibody was administered at 80 mg/mouse (3.2 mg/kg corresponding to 192 mg/60 kg), which was confirmed to be ineffective alone in a preliminary experiment. To a human administered with the NKT cell ligand-containing liposome composition, the immune checkpoint inhibitor may be administered at 480 mg per dose at intervales of 4 weeks or may be intravenously infused at 240 mg per dose, which is determined as a typical dose for adults, at intervale of 2 weeks.

The antitumor agent of the present invention can be safely administered to humans or non-human mammals (e.g., mice, rats, rabbits, dogs, cats, cattle, horses, monkeys, pigs).

The present invention also includes embodiments (1) and (2) below.
(1) A composition for enhancing the therapeutic effect of an immune checkpoint inhibitor, the composition including an NKT cell ligand-containing liposome.
(2) An agent for enhancing the therapeutic effect of an NKT cell ligand-containing liposome composition, the agent including an immune checkpoint inhibitor. In embodiments (1) and (2), the definition, examples, and methods for production (or acquisition) of the NKT cell ligand, the liposome, the NKT cell ligand-containing liposome composition, and the immune checkpoint inhibitor are the same as those described above for the combination drug of the present invention. In embodiments (1) and (2), the method for administration and dosage of the NKT cell ligand-containing liposome composition and the immune checkpoint inhibitor, the administration subject, and the therapeutic effect of the combination thereof may be appropriately selected or determined according to those described above in the section "5. Combination of Immune Checkpoint Inhibitor and NKT Cell Ligand-Containing Liposome Composition" for the combination drug of the present invention.

   The present invention also includes embodiments (3) and (4) below.
(3) An agent for enhancing immune checkpoint molecule expression by activating a population of anticancer immune cells, the agent including an NKT cell ligand-containing liposome composition.
(4) An agent for enhancing expression of an immune checkpoint ligand molecule on a cancer cell by artificially activating NKT cells, the agent including an NKT cell ligand-containing liposome composition. In embodiments (3) and (4), the definition, examples, and methods for production (or acquisition) of the NKT cell ligand, the liposome, and the NKT cell ligand-containing liposome composition are the same as those described above for the combination drug of the present invention.

### 2. Second Mode of the Invention

Hereinafter, the second mode of the present invention will be described in detail. The present invention is directed to a liposome composition including an NKT cell ligand and an antigen.

### [1. NKT Cell Ligand]

In the second mode of the present invention, the NKT cell ligand may be the same as that described above for the first mode of the present invention.

### [2. Antigen]

In the present invention, the antigen is a substance recognizable by the immune system, for example, which may be a self-component (e.g., a cancer antigen) or a foreign component (e.g., a pathogen antigen). In the present invention, the antigen may be a cancer antigen recognizable by the immune system. The cancer antigen may be a self-component produced by cancer cells or any of variations thereof. The cancer antigen can be produced by peptide synthesis based on gene sequence data. The cancer antigen may also be WT-1 described in the literature Jpn J Clin Oncol 2010 40: (5) 377-387.

### [3. Liposome]

In the second mode of the present invention, the liposome composition may include liposomes containing the glycolipid described above and the antigen. In the second mode of the present invention, the liposome may be the same as that described above for the first mode of the present invention.

### [4. Liposome Composition Including NKT Cell Ligand and Antigen]

The liposome composition of the present invention may include liposomes each including the NKT cell ligand and the antigen (e.g., the cancer antigen).

Such liposomes constituting the liposome composition of the present invention including the NKT cell ligand and the antigen may be prepared using known methods. For example, such methods are described in Liposome Technology, Vol. 1, 2nd Edition (by Gregory Gregoriadis (CRC Press, Boca Raton, Ann Arbor, London, Tokyo), Chapter 4, pp.67-80, Chapter 10, pp.167-184, and Chapter 17, pp.261-276 (1993). More specifically, such methods include, but are not limited to, ultrasonication, ethanol injection, French press, ether injection, cholate method, calcium-induced fusion, freeze-thaw, and reverse-phase evaporation. Specifically, the liposome composition including an NKT cell ligand and a cancer antigen may be produced as described below. As shown in the section "Examples", liposome components and the NKT cell ligand may be dissolved in an organic solvent in a glass test tube. Nitrogen gas may be blown to the solution so that the organic solvent can be removed by evaporation. The resulting residue may be completely dried in a desiccator (under reduced pressure). The dried product may be mixed with a solution of a cancer antigen in an isotonic aqueous solvent (e.g., 9% sucrose aqueous solution, 5% glucose aqueous solution, phosphate-buffered saline), and the mixture may be emulsified using a homogenizer to form liposomes containing the NKT cell ligand and the cancer antigen (the liposome composition of the present invention). The liposomes may further be passed through a polycarbonate membrane filter (with a pore size of 0.4 µm or 0.2 µm) at increased temperature and pressure to give uniform-sized liposomes. In general, finally, the liposomes may also be passed through a sterilizing filter (e.g., PVDF membrane filter with a pore size of 0.22 µm) for reliable sterility.

The liposome composition of the present invention including the NKT cell ligand and the antigen is preferably a liposome composition including RK-163 and an antigen, a liposome composition including RCAI-61 and an antigen, or a liposome composition including RCAI-137 and an antigen. More preferably, the liposome in each of these liposome compositions is Liposome #7 described above. The liposome composition of the present invention including the NKT cell ligand and the cancer antigen is preferably a liposome composition including RK-163 and a cancer antigen, a liposome composition including RCAI-61 and a cancer antigen, or a liposome composition including RCAI-137 and a cancer antigen. More preferably, the liposome in each of these liposome compositions is Liposome #7 described above.

In the liposome composition of the present invention including the NKT cell ligand and the antigen, the ratio of the weight of the glycolipid selected from the group consisting of the compound of Formula (I), the compound of Formula (II), and salts of the foregoing compounds (the NKT cell ligand according to the present invention) to the total weight of other lipids (components other than the NKT cell ligand (main component)) constituting the liposomes is typically 1:1-100,000, preferably 1:1-10,000, more preferably 1:1-1,000, even more preferably 1:1-100.

As a non-limiting example, the liposome composition of the present invention including the NKT cell ligand and the antigen typically has an average particle size of 10 to 1,000 nm, preferably 10 to 500 nm, more preferably 50 to 500 nm. The average particle size can be measured using conventional quasi-elastic light scattering.

In the present invention, the liposome composition of the present invention including the NKT cell ligand and the antigen may be formulated alone into a preparation, or the liposome composition including the NKT cell ligand and the antigen may be formulated together with a conventional pharmaceutically-acceptable additive (e.g., solvent, stabilizer, isotonic agent, analgesic, buffer, pH adjuster) into a preparation. The liposome composition of the present invention may be a liquid preparation, such as an injection. In such a case, the liquid preparation may be cryopreserved or may be stored after being subjected to water removal by freeze drying. The freeze-dried preparation may be mixed with distilled water for injection and dissolved again for use.

The liposome composition of the present invention including the NKT cell ligand and the antigen (e.g., cancer antigen) is useful as a pharmaceutical for inducing immune memory. The liposome composition of the present invention including the NKT cell ligand and the cancer antigen is useful as an antitumor agent. The liposome composition of the present invention including the NKT cell ligand and the cancer antigen is expected to be able to prophylactically induce activation and proliferation of a population of immune cells capable of recognizing a cancer antigen even in the absence of cancer. The liposome composition of the present invention including the NKT cell ligand and the cancer antigen is also expected to be able to prophylactically induce anticancer immune memory even in the absence of cancer. The liposome composition of the present invention including the NKT cell ligand and the cancer antigen is further expected to be able to exhibit an enhanced preventive effect against the development, recurrence, or metastasis of cancer.

The liposome composition including the NKT cell ligand and the cancer antigen is expected to be prophylactically effective against cancer, specifically hereditary cancer or its equivalent. Major examples include, but are not limited to, breast cancer, colorectal cancer, ovarian cancer, bone and soft tissue tumors, skin cancer, urological cancer, brain tumors, and endocrine system tumors; and other types of cancers, such as lung cancer, prostate cancer, esophageal cancer, gastric cancer, liver cancer, biliary tract cancer, spleen cancer, kidney cancer, bladder cancer, uterine cancer, testicular cancer, thyroid cancer, pancreatic cancer, brain tumors, skin cancer, melanoma, and hematologic tumors. The composition according to the present invention is also expected to be effective for subjects suffering from multiple cancers at the same time.

Examples of methods for administering the composition include, but are not limited to, intravenous administration, arterial administration, intramucosal administration, intranodal administration, and administration into affected tissues.

The NKT cell ligand of the liposome composition including the NKT cell ligand and the antigen may be administered in a dose sufficient to activate NKT cells, to induce activation and proliferation of antigen specific CD8T cells, and to form long-term immune memory for disease prevention. Typically, one cycle may include administering the liposome composition in an initial dose including 400 ng/kg to 4 mg/kg of the NKT cell ligand and 2 mg/kg to 20 mg/kg of the antigen and administering it again in the same dose after an interval of 4 weeks. It may vary depending on the type of the antigen how immune memory can be established and maintained. Thus, the initial cycle may be followed by follow-up for monitoring the number of CD8 immune memory T cells and followed by continuously administering the liposome composition at intervals of 4 weeks for the purpose of keeping the number of memory cells higher than that before the treatment. The administration may be performed multiple times depending on the condition of the subject or the severity of the disease.

Before the in vivo development of cancer cells, the liposome composition of the present invention including the NKT cell ligand and the cancer antigen can activate various immune cell populations and induce anticancer immune memory to prevent the development, recurrence, or metastasis of cancer. Simple change of the type of the antigen allows the liposome composition of the present invention including the NKT cell ligand and the antigen to be used not only for cancer prevention but also for infection prevention.

The liposome composition of the present invention including the NKT cell ligand and the antigen can be safely administered to humans or non-human mammals (e.g., mice, rats, rabbits, dogs, cats, cattle, horses, monkeys, pigs).

Hereinafter, the present invention will be described in more detail with reference to examples and test examples, which are not intended to limit the present invention.

### EXAMPLES

Examples were performed using RK-163, RCAI-61, and RCAI-137, which are the compounds of the chemical structural formulas shown above. Examples were performed using ovalbumin (OVA) as a surrogate cancer antigen. The surrogate cancer antigen is an artificially-introduced exogenous substance to which the immune system can respond. When expressed in cancer cell lines, the surrogate cancer antigen can behave like a cancer antigen. Herein, abbreviations are used as follows.
HSPC: Hydrogenated soybean phosphatidylcholine
DOPS: 1,2-Dioleoyl-sn-glycero-3-phospho-L-serine
OVA: Ovalbumin
PBS: Phosphate buffered saline

### [Example 1]

### [Preparation of NKT Cell Ligand-Containing Liposomes]

NKT cell ligand-containing liposome composition samples (liposome compositions containing Liposome #7 (shown above) and an NKT cell ligand (RK-163, RCAI-61, or RCAI-137) encapsulated in Liposome #7) for safety testing and primary pharmacology study were prepared by the methods shown below using the reagents and components shown in Table 3.

### (Composition of Prepared Samples)

### • For Safety Testing

15 ng/mL RK-163 (or RCAI-61 or RCAI-137), 220.8 ng/mL total lipids in 9% sucrose aqueous solution
Lipid composition: HSPC 103 ng/mL, DOPS 79.7 ng/mL, cholesterol 38.1 ng/mL

### • For Primary Pharmacology Study

1.5 ng/mL RK-163 (or RCAI-61 or RCAI-137), 22.08 ng/mL total lipids in 9% sucrose aqueous solution
Lipid composition: HSPC 10.3 ng/mL, DOPS 7.97 ng/mL, cholesterol 3.81 ng/mL
Particle size: 145.7 nm, PDI: 0.06813

**[Table 3]**

| | Manufacturer or preparation method | Cat No. | Lot No. |
|---|---|---|---|
| Chloroform | Wako Pure Chemical Industries, Ltd. | 038-02606 | ESL3428 |
| Methanol | Wako Pure Chemical Industries, Ltd. | 130-16585 | APF2160 |
| RK-163, RCAI-61, OR RCAI-137 | Provided by RIKEN | | F-01 |
| HSPC | Lipoid | LIPOID S PC-3 Batch No.525600-2190668-01/023 | |
| DOPS | Nippon Fine Chemical Co., Ltd. | - | RGF-NF 231 |
| Cholesterol | Kanto Chemical Co., Inc. | 07331-30 | 901B2144 |
| 9% sucrose aqueous solution | It was prepared by dissolving sucrose at 9% in ultrapure water and passing the solution through a sterilizing filter with a pore size of 0.22 µm._{∘} | | |

### (Preparation Methods)

### • For Safety Testing

To a flask were added 10.3 mg of HSPC, 7.97 mg of DOPS, and 3.81 mg of cholesterol and dissolved in 10 mL of methanol : chloroform (1:2 volume ratio) solution. A mixture was prepared of 1.0 mL of the resulting solution and 1.5 mL of a methanol : chloroform (1:2) solution of 100 µg/mL of RK-163 (or RCAI-61 or RCAI-137). While being heated, the resulting mixture was dried by nitrogen blow. The dried product was allowed to stand in a desiccator. To the dried product was added 10 mL of a 9% sucrose aqueous solution. The resulting mixture was emulsified using a Teflon^{®} homogenizer. The resulting emulsion was subjected to particle size regulation using an extruder (heated at 50°C and equipped with one polycarbonate membrane filter with a pore size of 0.2 µm). The resulting liposome dispersion was further diluted 1,000-fold with a 9% sucrose aqueous solution. The dilution was filtered through a sterilizing PVDF membrane filter with a pore size of 0.22 µm and then placed and stored at 4°C in a screw-capped glass bottle. The resulting solution sample was used for safety testing.

### • For Primary Pharmacology Study

A 1 mL aliquot of the sample for safety testing was added to another glass bottle and mixed and diluted with 9 mL of a 9% sucrose aqueous solution. The resulting liposome dispersion was filtered through a sterilizing PVDF membrane filter with a pore size of 0.22 µm and then placed and stored at 4°C in a screw-capped glass bottle. The resulting solution sample was used for primary pharmacology study. The resulting sample was an NKT cell ligand-containing liposome composition, which was used in Test Examples 1 to 3 and 5 as shown below.

### Test Example 1: Administration of NKT Cell Ligand (RK-163, RCAI-61, or RCAI-137)-Containing Liposome Composition for Activation of Immune Cell Population and for Expression of PD-1

An effective amount (1 ng/mouse) of the NKT cell ligand-containing liposome composition (RK-163-containing liposome composition (hereinafter referred to as RK-163-LIPO), RCAI-61-containing liposome composition (hereinafter referred to as RCAI-61-LIPO), or RCAI-137-containing liposome composition (hereinafter referred to as RCAI-137-LIPO)) and a surrogate tumor antigen (2 × 10⁷ of OVA-pulsed spleen cells) were administered to C57BL/6 mice (10 weeks old, 5 mice per group) via tail vein injection. The expression of PD-1 was determined and used as an indicator to analyze the influence on the innate immune system and the acquired immune system cell population by the adjuvant effect of NKT cell activation in the mouse liver. In a control group, the same amount of PBS was administered to the mice instead of the NKT cell ligand-containing liposome composition. The results are shown in FIG. 1. The results showed that RK-163-LIPO, RCAI-61-LIPO, and RCAI-137-LIPO activated NKT cells in the liver and consequently activated CD4 T cells and CD8 T cells in the acquired immune system and NK cells in the innate immune system, and showed an increase in the number of cells expressing PD-1 (an indicator of the activation). The administration of the NKT cell ligand-containing liposome composition to cancer-bearing mouse models (subcutaneous tumor models) was found to enhance the expression of PD-1 in various immune cell populations.

### Test Example 2: Enhancement of Expression of Immune Checkpoint Ligand Molecule (PD-1 Ligand Molecule: PD-L1) on In Vivo Tumor Cells by Administration of NKT Cell Ligand (RK-163, RCAI-61, or RCAI-137)-Containing Liposome Composition

An effective amount (1 ng/mouse) of the NKT cell ligand-containing liposome composition (RK-163-LIPO, RCAI-61-LIPO, or RCAI-137-LIPO) and 1 × 10⁶ of X-ray irradiated MO4 tumor cells (B16 mouse melanoma cells) were administered to C57BL/6J mice (10 weeks old, 5 mice per group) via tail vein injection. Subsequently, 3 × 10⁵ of living MO4 tumor cells were subcutaneously implanted into the mice. After 17 days, the expression of the immune checkpoint ligand molecule (PD-1 ligand molecule: PD-L1) by the tumor cells (MO4) was analyzed. In a control group, the same amount of PBS was administered to the mice instead of the NKT cell ligand-containing liposome composition. The results are shown in FIG. 2. The results showed a significant increase in the number of tumor cells expressing the immune checkpoint ligand molecule (PD-1 ligand: PD-L1) on the tumor cell (MO4) membrane in the mice administered with RK-163-LIPO, RCAI-61-LIPO, or RCAI-137-LIPO. The administration of the NKT cell ligand-containing liposome composition to cancer-bearing mouse models was found to enhance the expression of the immune checkpoint ligand molecule (PD-1 ligand molecule: PD-L1) on the tumor cells.

### Test Example 3: Enhancement of Expression of PD-1 Ligand Molecule (PD-L1) on Tumor Cells (MO4) by Artificial Activation of NKT Cells

An effective amount (1 ng) of the NKT cell ligand (RK-163, RCAI-61, or RCAI-137)-containing liposome composition (RK-163-LIPO, RCAI-61-LIPO, or RCAI-137-LIPO) and 3 × 10⁵ of 2E10 cells (NKT cell line) were subjected to mixed culture for 24 hours, which was followed by mixed culture with 2E10 cells (3 × 10⁵) and MO4 cells (3 × 10⁵). After 4 hours, 24 hours, and 48 hours, only the MO4 cells were retrieved and subjected to the measurement of the expression of the immune checkpoint ligand molecule (PD-1 ligand molecule: PD-L1). The results are shown in FIG. 3. The results showed a significant increase in the number of tumor cells (MO4) expressing the immune checkpoint ligand molecule (PD-1 ligand molecule: PD-L1) 24 to 48 hours after the mixed culture in all of the RK-163-LIPO, RCAI-61-LIPO, and RCAI-137-LIPO groups. For a study on the mechanism of PD-L1 expression enhancement on tumor cells, an NKT cell line (2E10) activated with the NKT cell ligand-containing liposome composition was subjected to mixed culture with tumor cells (MO4). The expression of the immune checkpoint ligand molecule PD-L1 on the tumor cells was monitored over time. As a result, the PD-L1 expression was found to reach maximum 24 to 48 hours after the start of the mixed culture.

### Test Example 4: IFNγ-Induced Enhancement of Immune Checkpoint Ligand Molecule PD-L1 Expression on Tumor Cells (MO4)

Tumor cells (MO4) (3x10⁵ cells) were cultured with a medium supplemented with 1 ng of recombinant IFNγ protein. At 0, 4, 24, and 48 hours, only the MO4 cells were retrieved and subjected to the measurement of the expression of the immune checkpoint ligand molecule (PD-1 ligand molecule: PD-L1). The results are shown in FIG. 4. The results showed a significant increase in the expression of the immune checkpoint ligand molecule (PD-1 ligand molecule: PD-L1) on the tumor cells 24 hours and 48 hours after the supplemental culture and thus revealed that IFNγ was involved in the expression of the immune checkpoint ligand molecule (PD-1 ligand molecule: PD-L1) on the tumor cells. Tumor cells (MO4) were cultured together with 1 ng/mL of recombinant IFNγ protein and retrieved over time for the analysis of PD-L1 expression. As a result, 24 to 48 hours after the start of culture, a significant increase in the expression of the PD-L1 ligand molecule on the tumor cells was observed, which showed that NKT cell-derived IFNγ enhanced the expression of the immune checkpoint ligand molecule (PD-1 ligand molecule: PD-L1) on the tumor cells in Test Example 2 (FIG. 2) and Test Example 3 (FIG. 3).

### Test Example 5: Induction of Strong Antitumor Effect by Combination Therapy with Anti-PD-1 Antibody and NKT Cell Ligand (RK-163, RCAI-61, or RCAI-137)-Containing Liposome Composition

The NKT cell ligand-containing liposome composition (RK-163-LIPO, RCAI-61-LIPO, or RCAI-137-LIPO) (at a dose of 1 ng/25 g body weight based on RK-163, RCAI-61, or RCAI-137) and 1 × 10⁶ of X-ray (50 Gy) irradiated B16 mouse melanoma cells (MO4) were intravenously administered to C57BL/6 mice (10 weeks old). Subsequently, 3 × 10⁵ of living B16 mouse melanoma cells (MO4) were subcutaneously implanted into the mice. The tumor growth was observed over time. On days 3 and 14 post implantation, 80 mg of the anti-PD-1 antibody was intravenously administered to the mice. The tumor diameter was measured on days 7, 10, 14, and 17 post the subcutaneous implantation of MO4 cells. All measurements were performed using a digital caliper. The tumor volume (mm³) was calculated by the equation: tumor volume (mm³) = longest diameter (mm) × shortest diameter (mm) × height (mm). The results are shown in FIGS. 5-1, 5-2, and 5-3. The results showed that the combined administration of the NKT cell ligand-containing liposome composition (RK-163-LIPO, RCAI-61-LIPO, or RCAI-137-LIPO) and the anti-PD-1 antibody had an antitumor effect stronger than that of the administration of each of them alone. The combined administration of the NKT cell ligand-containing liposome composition and the anti-PD-1 antibody to cancer-bearing mouse models (subcutaneous tumor models) was found to have an antitumor effect higher than that of the administration of each of them alone.

### [Example 2]

### [Preparation of Liposome Compositions Containing NKT Cell Ligand and Cancer Antigen]

Liposome composition samples for safety testing and primary pharmacology study each containing an NKT cell ligand and a cancer antigen (liposome compositions each containing Liposome #7 (shown above), and an NKT cell ligand (RK-163, RCAI-61, or RCAI-137) and a surrogate cancer antigen (OVA) encapsulated in Liposome #7) were prepared by the methods shown below using the reagents and components shown in Table 4.

### (Composition of Prepared Samples)

### • For Safety Testing

15 ng/mL RK-163 (or RCAI-61 or RCAI-137), 221 ng/mL lipids in 9% sucrose aqueous solution
Lipid composition: HSPC 103 ng/mL, DOPS 79.7 ng/mL, cholesterol 38.1 ng/mL
OVA: 100 mg/mL in 9% sucrose aqueous solution

### • For Primary Pharmacology Study

1.5 ng/mL RK-163 (or RCAI-61 or RCAI-137), 22.08 ng/mL lipids in 9% sucrose aqueous solution
Lipid composition: HSPC 10.3 ng/mL, DOPS 7.97 ng/mL, cholesterol 3.81 ng/mL
OVA: 10 mg/mL in 9% sucrose aqueous solution
Particle size: 145.7 nm, PDI: 0.06813

**[Table 4]**

| | Manufacturer or preparation method | Cat No. | Lot No. |
|---|---|---|---|
| Chloroform | Wako Pure Chemical Industries, Ltd. | 038-02606 | ESL3428 |
| Methanol | Wako Pure Chemical Industries, Ltd. | 130-16585 | APF2160 |
| RK-163, RCAI-61, OR RCAI-137 | Provided by RIKEN | | |
| HSPC | Lipoid | LIPOID S PC-3 Batch No.525600-2190668-01/023 | |
| DOPS | Nippon Fine Chemical Co., Ltd. | - | RGF-NF 231 |
| Cholesterol | Kanto Chemical Co., Inc. | 07331-30 | 901B2144 |
| 9% sucrose aqueous solution | It was prepared by dissolving sucrose at 9% in ultrapure water and passing the solution through a sterilizing filter with a pore size of 0.22 µm.ₒ | | |
| OVA | Wako Pure Chemical Industries, Ltd. | | LKP3544 |

### (Preparation Methods)

### • For Safety Testing

To a flask were added 10.3 mg of HSPC, 7.97 mg of DOPS, and 3.81 mg of cholesterol and dissolved in 10 mL of methanol : chloroform (1:2 volume ratio) solution. A mixture was prepared of 1.5 mL of the resulting solution and 1 mL of a methanol : chloroform (1:2) solution of 100 µg/mL of RK-163 (or RCAI-61 or RCAI-137). While being heated, the resulting mixture was dried by nitrogen blow to form a lipid film, which was allowed to stand in a desiccator. To the dried lipid film were added 2.208 mL of a 100 mg/mL OVA solution (in 9% sucrose aqueous solution) (resulting in a lipid concentration of 5 mg/mL). The resulting mixture was subjected to particle size regulation using an extruder (heated at 40°C, with a pore size of 0.2 µm (instead of 0.4 µm)). The unencapsulated OVA was removed by stirred ultrafiltration (membrane molecular weight cut-off: 300 kDa, retentate: 9% sucrose aqueous solution). In a clean bench, the filtrate was filtered through a sterilizing PVDF membrane filter with a pore size of 0.22 µm to yield size-regulated liposomes. The resulting solution was placed in a screw-capped glass bottle. The solution was further diluted 1000-fold with a 9% sucrose aqueous solution. The dilution was filtered through a 0.22 µm filter and placed and stored at 4°C in a screw-capped glass bottle. The resulting solution was a liposome composition sample (for safety testing) containing the NKT cell ligand (RK-163, RCAI-61, or RCAI-137) and the cancer antigen (OVA).

### • For Primary Pharmacology Study

A 1 mL aliquot of the liposome composition sample (for safety testing) containing the NKT cell ligand (RK-163, RCAI-61, or RCAI-137) and the cancer antigen (OVA) was added to another glass bottle and mixed with 9 mL of a sucrose solution. The resulting solution was filtered through a 0.2 µm polycarbonate membrane filter and then placed and stored at 4°C in a screw-capped glass bottle. The solution was a liposome composition sample (for primary pharmacology study) containing the NKT cell ligand (RK-163, RCAI-61, or RCAI-137) and the cancer antigen (OVA). The liposome composition sample containing the NKT cell ligand and the cancer antigen was used in Test Examples 6 to 10 as shown below.

### Test Example 6: Antitumor Activity (Dose) and ED50 of Liposome Composition Containing NKT Cell Ligand (RK-163) and Surrogate Cancer Antigen (OVA) (Hereinafter Abbreviated as RK-LIPO-OVA)

RK-LIPO-OVA (in a dose of 0.1 ng to 10 ng/25 g body weight based on RK-163) was intravenously administered to naive normal C57BL/6 mice (10 weeks old). Subsequently, 3 × 10⁵ of B16 mouse melanoma cells (MO4) expressing the surrogate cancer antigen OVA were subcutaneously implanted into the mice administered with RK-LIPO-OVA. The tumor diameter was measured on days 7, 11, 14, and 17 post implantation. All measurements were performed using a digital caliper. The tumor volume was calculated by the equation: tumor volume (mm³) = longest diameter (mm) × shortest diameter (mm) × height (mm). In a control group, the same amount of PBS was administered to the mice instead of the NKT cell ligand-containing liposome composition. The results are shown in FIG. 6. The results showed that RK-LIPO-OVA had a strong antitumor effect and inhibited the proliferation of the melanoma tumor cells in proportion to the amount of RK-163. PK-LIPO-OVA had an effective dose of 1 ng (on RK-163 basis)/25 g mouse body weight to 10 ng (on RK-163 basis)/25 g mouse body weight and an ED50 of 0.7797. RK-LIPO-OVA was intravenously administered to normal C57BL/6 mice, which was followed by subcutaneous implantation of B16 mouse melanoma cells (MO4) expressing the surrogate cancer antigen OVA into the RK-LIPO-OVA-administered mice. As a result, RK-LIPO-OVA was found to have a strong antitumor effect and to inhibit the proliferation of the MO4 tumor cells in proportion to the amount of the NKT cell ligand.

### Test Example 7: Activation and Proliferation of NKT Cells by Using Liposome Composition Containing NKT Cell Ligand (RK-163) and Surrogate Cancer Antigen (OVA) (Hereinafter Abbreviated as RK-LIPO-OVA)

An effective amount (10 ng (on RK163 basis)/mouse) of RK-LIPO-OVA was administered to normal C57BL/6 mice (10 weeks old, 5 mice per group) via tail vein injection and allowed to enter antigen-presenting cells (e.g., Kuppfer cells) in the liver so that the RK-163 ligand was presented as an antigen on the CD1d molecule to artificially activate NKT cells in the liver. As a result, the NKT cells expressed KLRG1 (activation marker) on the cell surface and produced IFNγ (an adjuvant substance that activates a population of other immune cells). In a control group, the same amount of PBS was administered to the mice instead of the NKT cell ligand-containing liposome composition. The results are shown in FIG. 7 (showing data on day 3 post RK-LIPO-OVA administration). RK-LIPO-OVA activated and expanded NKT cells. RK-LIPO-OVA also induced the expression of KLRG1 (activation marker) and the production of IFNγ. The administration of RK-LIPO-OVA to normal C57BL/6 mice via tail vein injection was found to artificially activate and expand NKT cells in the liver.

### Test Example 8: Activation of Innate Immune System Cell Population and Acquired Immune System Cell Population by Artificial Activation of NKT Cells Resulting from Administration of Liposome Composition Containing NKT Cell Ligand (RK-163) and Surrogate Cancer Antigen (OVA) (Hereinafter Abbreviated as RK-LIPO-OVA)

An effective amount (10 ng (on RK163 basis)/mouse) of RK-LIPO-OVA was administered to C57BL/6 mice (10 weeks old, 5 mice per group) via tail vein injection to artificially activate NKT cells in the liver. Analysis was performed on the influence on the innate immune system cell population and the acquired immune system cell population by the adjuvant effect of IFNγ production by the activated NKT cells in the liver. In a control group, the same amount of PBS was administered to the mice instead of the NKT cell ligand-containing liposome composition. The results are shown in FIG. 8. The results showed that the RK-LIPO-OVA administration induced artificial activation of NKT cells to allow the antigen (OVA) from RK-LIPO-OVA to induce proliferation of acquired immune system antigen (OVA)-specific CD4 T cells and CD8 T cells even in the normal mice. The results also showed that the artificial activation of NKT cells activated and expanded NK cells in the innate immune system. The intravenous administration of RK-LIPO-OVA to C57BL/6 mice was found to artificially activate NKT cells in the liver and consequently to induce proliferation of acquired immune system antigen (OVA)-specific CD4 T cells and CD8 T cells even in the normal mice and to expand NK cells in the innate immune system.

### Test Example 9: Establishment of Long-Term Immune Memory Using Liposome Composition Containing NKT Cell Ligand (RK-163) and Cancer Antigen (OVA) (Hereinafter Abbreviated as RK-LIPO-OVA)

An effective amount (10 ng/mouse) of RK-LIPO-OVA was intravenously administered to C57BL/6 mice (10 weeks old, 5 mice per group). On 60 day post the RK-LIPO-OVA administration, the mice were sacrificed, and spleen cells were analyzed. The results are shown in FIG. 9. The results showed the proliferation of a population of OVA-specific CD8 T cells and the formation of long-term immune memory CD8 T cells (OVA-specific memory CD8 T cells) on day 60 post the RK-LIPO-OVA administration, which means the formation and maintenance of long-term anticancer immune memory. The intravenous administration of RK-LIPO-OVA to C57BL/6 mice was found to generate surrogate antigen OVA-specific memory CD8 T cells on day 60 post the RK-LIPO-OVA administration, which means the formation and maintenance of long-term anticancer immune memory.

### Test Example 10: Antitumor Effect on Day 60 Post Establishment of Long-Term Immune Memory Using Liposome Composition Containing NKT Cell Ligand (RK-163) and Cancer Antigen (OVA) (Hereinafter Abbreviated as RK-LIPO-OVA)

Normal C57BL/6 mice were immunized with an effective amount (10 ng/mouse) of RK-LIPO-OVA and observed for 60 days without further treatment. The C57BL/6 mice having surrogate cancer antigen OVA-specific long-term memory induced therein were subcutaneously implanted with 3 × 10⁵ of B16 mouse melanoma cells (MO4) expressing the surrogate cancer antigen OVA. On 7, 11, 14, and 17 days post implantation, the tumor diameter was measured. All measurements were performed using a digital caliper. The tumor volume (mm³) was calculated by the equation: tumor volume (mm³) = longest diameter (mm) × shortest diameter (mm) × height (mm). In a control group, the same amount of PBS was administered to the mice instead of the NKT cell ligand-containing liposome composition. The results are shown in FIG. 10. The results showed that on day 60 post the RK-LIPO-OVA administration, the mice had immune memory induced against the surrogate cancer antigen OVA and thus exhibited significant suppression of the growth of the B16 mouse melanoma cells (MO4) expressing the surrogate cancer antigen OVA, which were implanted on day 60. In addition, the administration of the liposome composition RCAI-61-OVA containing the NKT cell ligand RCAI-61 or the liposome composition RCAI-137-OVA containing the NKT cell ligand RCAI-137 was also found to significantly suppress the growth of the B16 mouse melanoma cells (MO4) expressing the surrogate cancer antigen OVA. The results showed that RK-LIPO-OVA exhibited a beneficial antitumor effect in normal mice. RK-LIPO-OVA was administered to normal mice. On 60 day post administration, B16 mouse melanoma cells (MO4) expressing the surrogate cancer antigen OVA were implanted into the mice. The results showed significant suppression of the growth of the B16 mouse melanoma cells expressing the surrogate cancer antigen OVA, which were implanted on day 60 post the RK-LIPO-OVA administration. The results indicated that surrogate cancer antigen OVA-specific immune memory was established in normal mice to provide a beneficial antitumor effect.

### INDUSTRIAL APPLICABILITY

The present invention (the first mode) provides a preparation including a combination of an immune checkpoint inhibitor and an NKT cell ligand-containing liposome composition, which is useful for the treatment of various types of cancers, including carcinomas that are difficult to cure due to low expression of PD-L1 (PD-1 ligand molecule) and resistant to immune checkpoint inhibitors and cancers that are resistant to conventional NKT cell ligand-containing liposome compositions.

The present invention (the second mode) provides a liposome composition including an NKT cell ligand and an antigen (e.g., a cancer antigen, a pathogen antigen). The liposome composition may include an NKT cell ligand-containing material, which has the function of artificially activating NKT cells, and an antigen (e.g., a cancer antigen, a pathogen antigen). The liposome composition can activate a population of antigen-specific immune cells and induce long-term immune memory and thus provide a useful preparation for preventing diseases including cancers and infections.

The present application is based on Japanese Patent Application No. 2023-132368, the contents of which are incorporated herein by reference in their entirety.

## Claims

1. An antitumor agent comprising a combination of an immune checkpoint inhibitor and an NKT cell ligand-containing liposome composition.

2. The antitumor agent according to claim 1, wherein the NKT cell ligand is a glycolipid selected from the group consisting of a compound represented by Formula (I) below, a compound represented by Formula (II) below, and salts of the foregoing compounds, 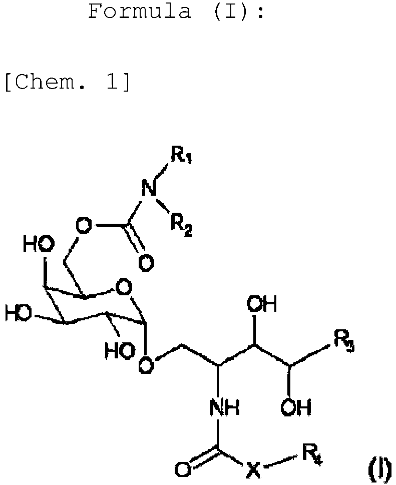
wherein X represents an alkylene group or -NH-,
R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with an adjacent nitrogen atom,
R₃ represents a hydrocarbon group having 1 to 20 carbon atoms, and
R₄ represents a hydrocarbon group having 1 to 30 carbon atoms, 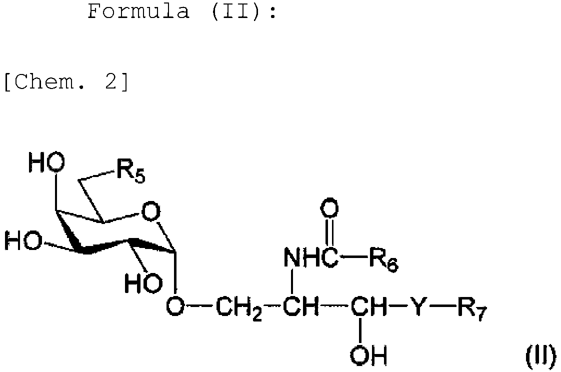
wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.

3. The antitumor agent according to claim 2, wherein the glycolipid is the compound represented by Formula (I) or a salt thereof.

4. The antitumor agent according to claim 3, wherein the glycolipid is a compound represented by the formula: 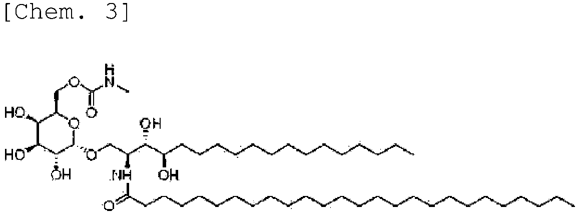 or a salt thereof.

5. An agent for enhancing immune checkpoint molecule expression by activating a population of anticancer immune cells, the agent comprising an NKT cell ligand-containing liposome composition.

6. An agent for enhancing expression of an immune checkpoint ligand molecule on a cancer cell by artificially activating NKT cells, the agent comprising an NKT cell ligand-containing liposome composition.

7. A composition for enhancing a therapeutic effect of an immune checkpoint inhibitor, the composition comprising an NKT cell ligand-containing liposome.

8. An agent for enhancing a therapeutic effect of an NKT ligand-containing liposome composition, the agent comprising an immune checkpoint inhibitor.

9. A method of treating a malignant tumor in a subject, the method comprising administering an immune checkpoint inhibitor and an NKT cell ligand-containing liposome composition to the subject.

10. The method according to claim 9, wherein the NKT cell ligand is a glycolipid selected from the group consisting of a compound represented by Formula (I) below, a compound represented by Formula (II) below, and salts of the foregoing compounds, 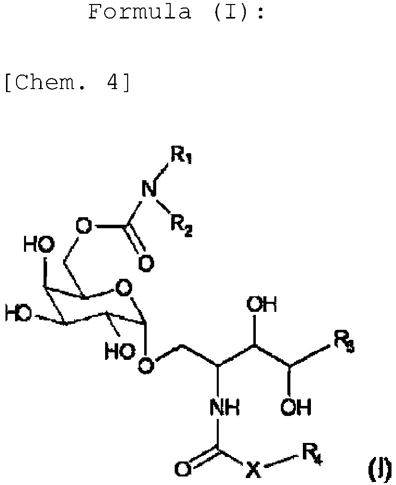
wherein X represents an alkylene group or -NH-,
R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with an adjacent nitrogen atom,
R₃ represents a hydrocarbon group having 1 to 20 carbon atoms, and
R₄ represents a hydrocarbon group having 1 to 30 carbon atoms, 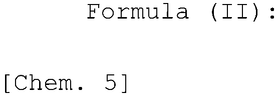 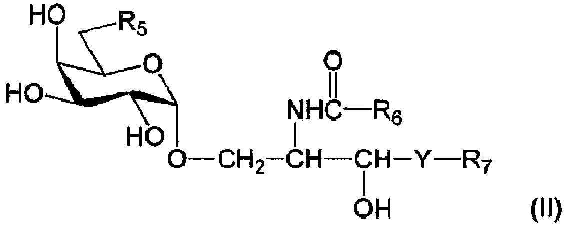
wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.

11. The method according to claim 10, wherein the glycolipid is the compound represented by Formula (I) or a salt thereof.

12. The method according to claim 11, wherein the glycolipid is a compound represented by the formula: 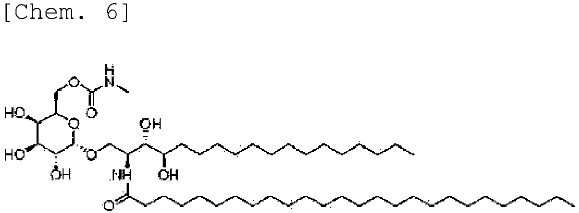 or a salt thereof.

13. A method of enhancing immune checkpoint molecule expression in a subject, the method comprising administering, to the subject, an NKT cell ligand-containing liposome composition to activate a population of anticancer immune cells.

14. A method of enhancing expression of an immune checkpoint ligand molecule on a cancer cell in a subject, the method comprising administering, to the subject, an NKT cell ligand-containing liposome composition to artificially activate NKT cells in the subject.

15. A pharmaceutical for use in malignant tumor treatment, the pharmaceutical comprising a combination of an immune checkpoint inhibitor and an NKT cell ligand-containing liposome composition.

16. The pharmaceutical according to claim 15, wherein the NKT cell ligand is a glycolipid selected from the group consisting of a compound represented by Formula (I) below, a compound represented by Formula (II) below, and salts of the foregoing compounds, 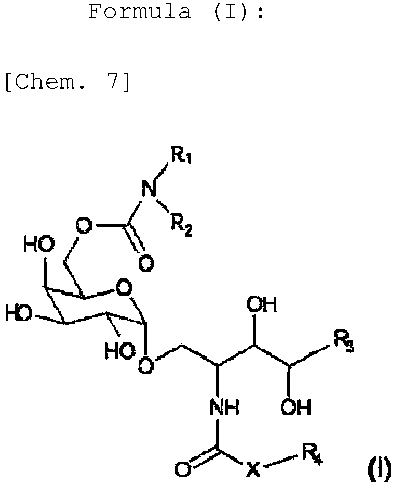
wherein X represents an alkylene group or -NH-,
R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with an adjacent nitrogen atom,
R₃ represents a hydrocarbon group having 1 to 20 carbon atoms, and
R₄ represents a hydrocarbon group having 1 to 30 carbon atoms, 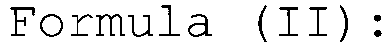 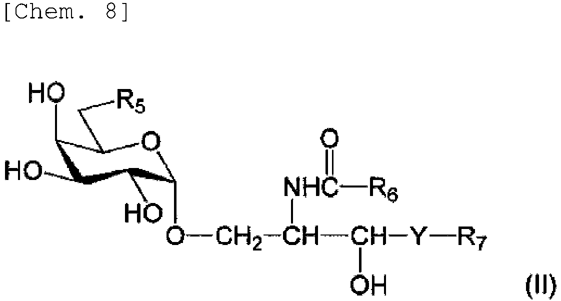
wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.

17. The pharmaceutical according to claim 16, wherein the glycolipid is the compound represented by Formula (I) or a salt thereof.

18. The pharmaceutical according to claim 17, wherein the glycolipid is a compound represented by the formula: 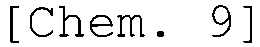 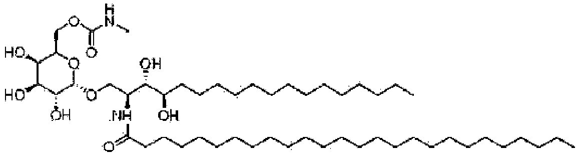 or a salt thereof.

19. A composition for use in enhancing immune checkpoint molecule expression by activating a population of anticancer immune cells, the composition comprising an NKT cell ligand-containing liposome.

20. A composition for use in enhancing expression of an immune checkpoint ligand molecule on a cancer cell by artificially activating NKT cells, the composition comprising an NKT cell ligand-containing liposome.

21. A composition for use in enhancing a therapeutic effect of an immune checkpoint inhibitor, the composition comprising an NKT cell ligand-containing liposome.

22. An agent for use in enhancing a therapeutic effect of an NKT cell ligand-containing liposome composition, the agent comprising an immune checkpoint inhibitor.

23. An composition for use as a pharmaceutical, the composition comprising: an immune checkpoint inhibitor; and an NKT cell ligand-containing liposome.

24. Use of a combination of an immune checkpoint inhibitor and an NKT cell ligand-containing liposome composition for manufacture of an antitumor agent.

25. The use according to claim 24, wherein the NKT cell ligand is a glycolipid selected from the group consisting of a compound represented by Formula (I) below, a compound represented by Formula (II) below, and salts of the foregoing compounds, 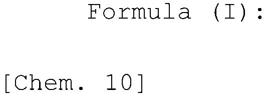 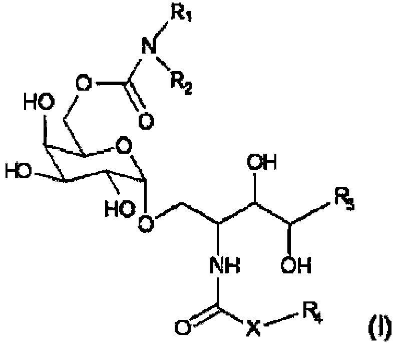
wherein X represents an alkylene group or -NH-,
R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with an adjacent nitrogen atom,
R₃ represents a hydrocarbon group having 1 to 20 carbon atoms, and
R₄ represents a hydrocarbon group having 1 to 30 carbon atoms, 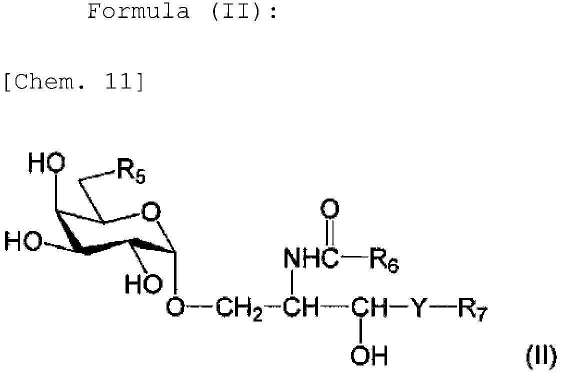
wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.

26. The use according to claim 25, wherein the glycolipid is the compound represented by Formula (I) or a salt thereof.

27. The use according to claim 26, wherein the glycolipid is a compound represented by the formula: 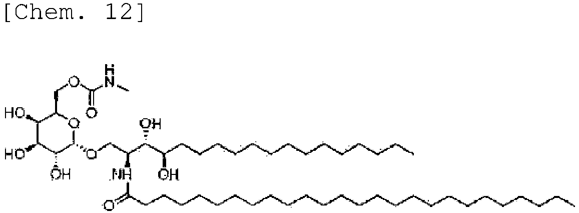 or a salt thereof.

28. Use of an NKT cell ligand-containing liposome composition for manufacture of an agent for enhancing immune checkpoint molecule expression by activating a population of anticancer immune cells.

29. Use of an NKT cell ligand-containing liposome composition for manufacture of an agent for enhancing expression of an immune checkpoint ligand molecule on a cancer cell by artificially activating NKT cells.

30. Use of an NKT cell ligand-containing liposome composition for manufacture of an agent for enhancing a therapeutic effect of an immune checkpoint inhibitor.

31. Use of an immune checkpoint inhibitor for manufacture of an agent for enhancing a therapeutic effect of an NKT cell ligand-containing liposome composition.

32. A pharmaceutical composition comprising a combination of an immune checkpoint inhibitor and an NKT cell ligand-containing liposome composition.

33. A liposome composition comprising: an NKT cell ligand; and an antigen.

34. The composition according to claim 33, wherein the NKT cell ligand is a glycolipid selected from the group consisting of a compound represented by Formula (I) below, a compound represented by Formula (II) below, and salts of the foregoing compounds, 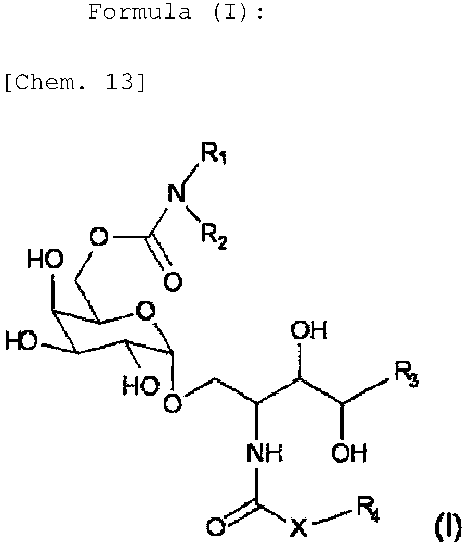
wherein X represents an alkylene group or -NH-,
R₁ and R₂ are the same or different and each represents a hydrogen atom, an alkyl group, a hydroxyl group, an alkoxy group, or an optionally-substituted aryl group, R₁ and R₂ may form a 5- or 6-membered ring together with an adjacent nitrogen atom,
R₃ represents a hydrocarbon group having 1 to 20 carbon atoms, and
R₄ represents a hydrocarbon group having 1 to 30 carbon atoms, 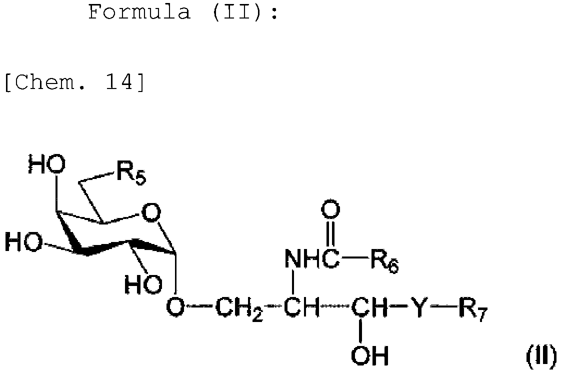
wherein R₅ represents a hydrogen atom, an alkyl group having 1 to 7 carbon atoms, an alkoxy group having 1 to 6 carbon atoms, or a halogen atom, R₆ and R₇ each independently represent a substituted or unsubstituted hydrocarbon group having 1 to 28 carbon atoms, and Y represents -CH₂-, -CH(OH)-, or -CH=CH-.

35. The composition according to claim 34, wherein the glycolipid is the compound represented by Formula (I) or a salt thereof.

36. The composition according to claim 35, wherein the glycolipid is a compound represented by the formula: 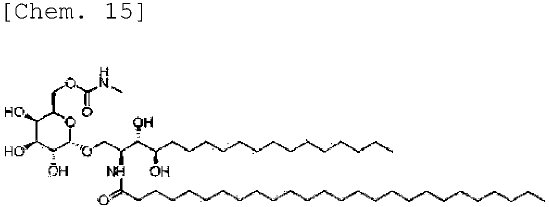 or a salt thereof.

37. The composition according to any one of claims 33 to 36, wherein the composition is for use in inducing immune memory.

38. The composition according to any one of claims 33 to 36, wherein the antigen is a cancer antigen.

39. The composition according to claim 38, wherein the composition is for use in prophylactically inducing, even in the absence of cancer, activation and proliferation of a population of immune cells capable of recognizing a cancer antigen.

40. The composition according to claim 38, wherein the composition is for use in prophylactically inducing anticancer immune memory even in the absence of cancer.

41. The composition according to claim 38, wherein the composition is for use in providing an enhanced preventive effect against development, recurrence, and metastasis of cancer.

42. A method of inducing immune memory in a subject, the method comprising administering the composition according to any one of claims 33 to 36 to the subject.

43. A method of prophylactically inducing, even in the absence of cancer, activation and proliferation of a population of immune cells capable of recognizing a cancer antigen in a subject, the method comprising administering the composition according to claim 38 to the subject.

44. A method of prophylactically inducing anticancer immune memory even in the absence of cancer in a subject, the method comprising administering the composition according to claim 38 to the subject.

45. A method of inhibiting development, recurrence, and metastasis of cancer in a subject, the method comprising administering the composition according to claim 38 to the subject.

46. The composition according to any one of claims 33 to 36, wherein the composition is for use in inducing immune memory.

47. The composition according to claim 38, wherein the composition is for use in prophylactically inducing, even in the absence of cancer, activation and proliferation of a population of immune cells capable of recognizing a cancer antigen.

48. The composition according to claim 38, wherein the composition is for use in inducing anticancer immune memory even in the absence of cancer.

49. The composition according to claim 38, wherein the composition is for use in providing an enhanced preventive effect against development, recurrence, and metastasis of cancer.

50. The composition according to any one of claims 33 to 36, wherein the composition is for use as a pharmaceutical.

51. Use of the composition according to any one of claims 33 to 36 for manufacture of an immune memory inducing agent.

52. Use of the composition according to claim 38 for manufacture of an agent for prophylactically inducing, even in the absence of cancer, activation and proliferation of a population of immune cells capable of recognizing a cancer antigen.

53. Use of the composition according to claim 38 for manufacture of an agent for prophylactically inducing anticancer immune memory even in the absence of cancer.

54. Use of the composition according to claim 38 for manufacture of an agent for inhibiting development, recurrence, and metastasis of cancer.
